# EUROPEAN PATENT SPECIFICATION

(11) **EP 4 458 393 B1**
(45) Date of publication and mention of the grant of the patent: **22.10.2025**
(21) Application number: 23181984.8
(22) Date of filing: 28.06.2023
(51) Int. Cl.: A61M 16/00, A61B 5/00, A61B 5/08, A61M 16/10

(54) **PAP SYSTEM OF PROVIDING DIAGNOSES USING THE SAME**
PAP-SYSTEM ZUR BEREITSTELLUNG VON DIAGNOSEN DAMIT
SYSTÈME PAP DE FOURNITURE DE DIAGNOSTICS L'UTILISANT

(30) Priority: 04.05.2023 US 202318143567
(43) Date of publication of application: 06.11.2024
(73) Proprietor: Khan, Macksoud, Palo Alto, CA 94303 (US)
(72) Inventor: Khan, Macksoud, Palo Alto, CA 94303 (US)
(74) Representative: Valet Patent Services Limited

(56) References cited:
- WO-A1-2021/176426
- WO-A1-2022/058930
- WO-A1-2022/070038
- WO-A1-2022/130205

## Description

### Background of the Invention

The present invention relates to a positive airway pressure (PAP) system for providing positive air pressure to a patient in order to treat sleep apnea. The three most common forms of PAP therapy are CPAP, APAP, and BiPAP. CPAP stands for "continuous positive airway pressure". CPAP systems deliver air to a patient at a single, constant pressure while the patient sleeps. CPAP is typically the first type of PAP that a patient will try when determining which type of PAP to use to treat sleep apnea. BiPAP stands for "bilevel positive airway pressure". BiPAP systems deliver air to a patient at two different pressures. BiPAP therapy typically provides a higher level of air pressure when a patient is inhaling and a lower level of air pressure when the patient is exhaling. APAP stands for "automatic positive airway pressure", or "auto-adjustable positive airway pressure". APAP therapy provides air to a patient at varying pressures depending on the airflow of the patient's exhale. If an APAP system senses that the airflow of a patient's exhale has decreased, it may provide air to the patient at a higher pressure when the patient is inhaling. Likewise, if an APAP system senses that the airflow of a patient's exhale has increased, it may provide air to the patient at a lower pressure when the patient is inhaling. Many APAP systems vary the pressure of the air delivered to the patient during the patient's inhale, and provide air to the patient at a single, constant pressure while the patient is exhaling.

PAP is commonly used to treat sleep apnea, which is the periodic starting and stopping of breathing during sleep. A common form of sleep apnea is "obstructive sleep apnea" (OSA), wherein a patient's throat muscles block the patient's airway, causing sleep apnea and snoring. Obstructive sleep apnea is a potentially life-threatening condition since it inhibits the patient's ability to breathe. Certain OSA is caused by the position in which the patient sleeps. This type of OSA is referred to as "positional obstructive sleep apnea", or "positional OSA".

OSA is typically diagnosed by a sleep study, also referred to as a "polysomnography", which may be abbreviated as "PSG". During a polysomnography, a patient's vital signs such as but not limited to pulse rate, body temperature, heart rate, respiration rate, blood pressure, and oxygen saturation level are measured and monitored over time. The measurements of the patient's vital signs are used to calculate an apnea-hypopnea index (AHI) for the patient, which is the combined average number of apneas and hypopneas that occur per hour of sleep. An apnea is an occurrence of little to no respiration activity. A hypopnea is an occurrence of respiration activity that is decreased from normal levels by at least 50%.

A polysomnography is typically performed using a plurality of sensors configured at various locations of a patient's body. These sensors may be connected to a processor via wires, and therefore may be bothersome to a patient during sleep. Furthermore, the presence of wires may allow the patient to accidentally break the connection between one or more of the sensors and the processor during sleep, thereby rendering the polysomnography useless since the data gathered by the sensors is not analyzed by the processor. Depending on what sensors are used and where they are placed on the patient's body, the sensors may also be bothersome to the patient during sleep, or may be accidentally detached from the patient by the patient's movement during sleep. WO2022/130205 describes a respiratory system comprising one or more sensors. The sensors can be used to determine one or more sleep related parameters.

### Summary of the Invention

The present invention is defined by the claims.

### Summary of the disclosure

The present disclosure relates to a PAP system that uses a mask with a PAP unit and air passage to provide positive air pressure to a patient. The PAP system, also referred to herein as "the system", may have a plurality of sensors, such as but not limited to a heart rate monitor, a pulse oximeter, a microphone, a gyroscope, a pressure sensor, and an ammeter. The PAP system may further have at least one ECG sensor. The plurality of sensors may obtain a plurality of measurements, which may be analyzed by a processor. The plurality of measurements may be stored in a memory of the PAP system. The memory may exist as a hard drive such as but not limited to a hard drive disk or solid-state hard drive. The processor and the memory may be configured within the PAP unit of the PAP system. The processor and/or memory may alternatively be configured within a device outside of the PAP system such as but not limited to a patient's smartphone, computer, or other device; or a medical professional's smartphone, computer, or other device.

The PAP unit of the PAP system may contain a blower fan that forces air through the air passage and into the mask. The blower fan may rotate about a blower fan axis. The rotation of the blower fan may cause the air to move through the air passage, into the mask, and into the patient's nose and/or mouth. The air passage may be configured between the PAP unit and the mask. The blower fan may be powered by one or more batteries, which may be configured within PAP unit. The blower fan may alternatively be powered by an electrical connection to a wall outlet.

The microphone may be configured on the mask or within the mask. In some embodiments, this may be an ideal location for the microphone since it is close to the patient's nose and mouth, and therefore can easily obtain sound measurements from the patient. The microphone may alternatively be configured on or within the PAP unit or the wearable article. In some embodiments, these may be the ideal locations for the microphone so that any standard PAP mask can be used with the PAP unit. The processor may analyze the sound measurements obtained by the microphone to detect if the patient is snoring. Snoring may be detected by determining if the sound measurements are outside of a normal range. A normal range of sound measurements for regular breathing may be 5-12 decibels. Snoring may be considered sound measurements that exceed 12 decibels.

The gyroscope may be configured on the mask or within the mask. **In** some embodiments, this may be the ideal location for the gyroscope since the goal of the gyroscope is to obtain position measurements (also referred to herein as "movement measurements") of the mask. The position measurements may be analyzed by the processor to determine how much the patient's head moves during sleep, and in what positions the patient's head is configured during sleep. This may aid the system in determining if the patient suffers from positional OSA.

The gyroscope may alternatively be configured on or within the wearable article. In some embodiments, this may be the ideal location for the gyroscope so that any standard PAP mask can be used with the PAP unit. In these embodiments, the gyroscope may obtain position measurements of the wearable article that may be caused by the patient while the patient sleeps. These position measurements may be analyzed by the processor to determine how much the body part of the patient on which the wearable article is worn moves while the patient sleeps, and in what positions that body part is configured while the patient sleeps. This may aid the system in determining if the patient suffers from positional OSA.

In some embodiments, the gyroscope and/or microphone may be configured onto or within the mask by the patient. These embodiments allow the PAP unit to be used with a standard "off-the-shelf" PAP mask, while still allowing the gyroscope and/or microphone to be configured on or within the mask.

The gyroscope may use electronic sensors to measure rotational acceleration of the mask about three perpendicular axes, which the processor may then use to calculate the amount of movement of the mask and the positions in which the mask has moved. The processor may then use this data together with measurements from the other sensors such as the heart rate monitor and pulse oximeter to determine if certain positions cause the patient to experience positional OSA. The processor may also determine if certain position measurements fall outside of a normal range of position measurements. A normal range of position measurements may be determined by the processor by analyzing the position measurements concurrently with the measurements of the other sensors to determine which position measurements are not associated with apneas or hypopneas.

The pressure sensor may be configured within the air passage. The pressure sensor may obtain pressure measurements of the air within the air passage. The processor may analyze the pressure measurements to detect a mask leak. A mask leak may be detected by the processor if the pressure measurements are below 4 cmH2O, below 3 cmH20, below 2cmH20, or any pressure value between and including the values provided.

The processor may also analyze the pressure measurements to calculate respiratory air flow of the patient. The processor may determine if the respiratory air low falls outside of a normal range. A normal range of respiratory air flow may be 0.1 cfpm (cubic feet per minute) to 0.4 cfpm, including the values provided. The processor may also determine if the respiratory air flow of the patient changes over time.

The pressure of the air passage of the mask may be increased by ensuring that the mask fits tightly over the patient's face. The mask may have cushions that are configured against the patient's face, and that deform to form a seal between the mask and the patient's face. The fit of the mask against the patient's face may be adjusted by providing a mask of a different size or by providing cushions of different sizes. The fit of the mask against the patient's face may also be adjusted by tightening straps that secure the mask to the patient's face. The pressure of the air passage of the mask may also be increased by increasing the speed at which the blower fan rotates, thereby drawing more air into the mask.

The ammeter may be configured within the PAP unit. The ammeter may obtain electrical current measurements of electrical current within PAP system. The PAP system may use one or more batteries or an electrical connection to a wall outlet to power the blower fan and other components of the system such as the various sensors and processor. Therefore, electrical currents are present within the PAP system. The processor may analyze the electrical current measurements to detect a mask leak. If the current of the PAP system is detected as being open, then the processor may determine that the mask has fallen off of the patient's face. If the current of the system is detected as steadily increasing, then the processor may determine that the mask is still on the patient's face but that a mask leak is present.

The PAP system may also have a wearable article. The wearable article may be a ring, bracelet, ankle bracelet, necklace, headband, sleeve, or any other article that may be worn on or around a body part of the patient. The wearable article may have one or more batteries that power the sensors and other components configured on the wearable article. The batteries of the wearable article provide the ability of the sensors configured on the wearable article to be used without wires to connect the sensors to an external power source. This allows the patient to be more comfortable when sleeping. This also allows the patient to move freely and naturally when sleeping, which allows the sensors to obtain accurate measurements of the patient's vital signs in order to provide an accurate diagnosis. Wearable articles such as rings may be non-invasive whereby the patient may not even notice that they are wearing the wearable article, which further allows the patient to be more comfortable when sleeping and to exhibit their natural sleeping behavior (which may include symptoms of OSA) that can be captured by the various measurements of the patient's vital signs by the various sensors of the system.

The heart rate monitor may be configured on the wearable article. The heart rate monitor may obtain heart rate measurements of the patient. The processor may analyze the heart rate measurements to determine if the heart rate measurements fall outside of a normal range. A normal range of heart rate measurements may be 30-60 bmp (beats per minute), including the values provided.

The pulse oximeter may be configured on the wearable article. The pulse oximeter may obtain oxygen saturation measurements of the patient's blood. The processor may analyze the oxygen saturation measurements to determine if the oxygen saturation measurements fall outside of a normal range. A normal range of oxygen saturation measurements may be 95% or greater.

The at least one ECG sensor may be configured on the wearable article. The at least one ECG sensor may alternatively be detachably attached to the patient's chest by adhesives or suction cups. In embodiments wherein the wearable article is a necklace, the at least one ECG sensor may be configured both on the wearable article and against the patient's chest by adhesives or suction cups. The at least one ECG sensor may obtain electrical activity measurements of the patient's heart. The processor may analyze the electrical activity measurements to calculate respiratory effort of the patient. The processor may determine if the respiratory effort of the patient falls outside of a normal range. A normal range of respiratory effort may be 12-16 breaths per minute, including the values provided.

The processor may alternatively use position measurements of the at least one ECG sensor ("ECG position measurements") to calculate the respiratory effort of the patient. This is called "ECG-Derived Respiration" (EDR). Positions of the at least one ECG sensor may be obtained by other sensors configured on the ECG sensors ("ECG position sensors"). The ECG position sensors may be any common position sensors such as but not limited to gyroscopes and/or accelerometers. The processor may analyze the ECG position measurements to measure the movement of the patient's chest while breathing, and thereby calculate the respiratory effort of the patient.

The processor may analyze the heart rate measurements, oxygen saturation measurements, sound measurements, position measurements, electrical activity measurements, pressure measurements, and electrical current measurements concurrently to determine a sleep diagnosis and/or to suggest treatment for the patient. Alternatively, only some of the measurements described herein may be analyzed concurrently by the processor to determine a sleep diagnosis and/or to suggest treatment for the patient.

When the various sensors of the PAP system obtain the various measurements, the measurements may be obtained in the form of non-transitory computer-readable media. In this manner, the measurements may be referred to as "data". The PAP system may have a transmitter that transmits the various measurements from the sensors to the processor. The transmitter may transmit the measurements wirelessly via wireless data transmission technology such as but not limited to Bluetooth or Wi-Fi. The transmitter may also transmit the measurements from the sensors to a patient's device such as but not limited to a smartphone, laptop, tablet, or personal computer. The transmitter may also transmit the measurements from the sensors to a medical professional's device such as but not limited to a smartphone, laptop, tablet, or personal computer, whereby a medical professional may analyze the measurements to provide a diagnosis or suggest treatment. The transmitter may also transmit the analyses performed by the processor to the patient's device and the medical professional's device.

The various components of the PAP system may be powered by a standard electrical wall outlet connection. Alternatively, the various components of the PAP system may be powered by one or more batteries. The one or more batteries may be configured within the PAP unit and/or within the wearable article. The PAP system may have one or more battery sensors that may each use a shunt to measure the current of a battery. The one or more battery sensors may use these measurements to determine the temperature and voltage of each battery. The one or more battery sensors may alert the patient if one or more of the batteries is low and needs to be recharged or replaced.

The normal ranges of the various measurements obtained and analyzed by the PAP system may be originally set to default ranges. Alternatively, these normal ranges may be set by the patient or by a medical professional. The normal ranges may be adjusted by the patient or by the medical professional at any time during use of the PAP system. Furthermore, in some embodiments, the PAP system may automatically adjust the normal ranges based on feedback provided by the patient or a medical professional. Feedback may be provided by the patient via a patient's device or by a medical professional via a medical professional's device in the form of non-transitory computer-readable media.

The PAP system may be a CPAP system, BiPAP system, or APAP system. The pressure of the air provided to the patient by the PAP system may be anywhere from 3 cmH20 to 25 cmH20, including the values provided. In embodiments wherein the PAP system is an APAP system, the pressure sensor may obtain measurements of the air pressure of the patient's exhale and transmit these measurements to the processor via the transmitter. The processor may then determine the proper air pressure of air to provide to the patient based on the air pressure of the patient's exhale. The processor may then provide instructions to the blower fan in the form of non-transitory computer-readable media of how fast to spin, thereby increasing or decreasing the air pressure of the air provided to the patient based on the air pressure of the patient's exhale.

A method is provided for a diagnosis and/or suggesting treatment using the PAP system described herein. The PAP system may be provided. The heart rate monitor may be used to obtain heart rate measurements of the patient. The pulse oximeter may be used to obtain oxygen saturation measurements of the patient's blood. The microphone may be used to obtain sound measurements of the patient. The gyroscope may be used to obtain position measurements of the patient. The pressure sensor may be used to obtain pressure measurements of the air within the air passage of the mask. The at least one ECG sensor may be used to obtain electrical activity measurements of the patient's heart. The ammeter may be used to obtain electrical current measurements of the PAP system. The at least one ECG position sensor may be used to obtain ECG position measurements of the at least one ECG sensor.

The processor may be used to calculate respiratory air flow of the patient using the pressure measurements. The processor may be used to calculate respiratory effort of the patient using the electrical activity measurements of the patient. The processor may alternatively be used to calculate respiratory effort of the patient using the ECG position measurements. The processor may be used to detect a mask leak using the electrical current measurements of the PAP system. The heart rate measurements, oxygen saturation measurements, pressure measurements, sound measurements, position measurements, electrical activity measurements, ECG position measurements, and electrical current measurements may be analyzed concurrently to provide a diagnosis and/or suggest treatment. Alternatively, only some of these measurements may be analyzed concurrently to provide a diagnosis and/or suggest treatment. The diagnosis may be a sleep diagnosis that is related to sleep-related conditions such as OSA.

Described is a method of performing a polysomnography using the PAP system described herein. The method of providing a diagnosis and/or suggesting treatment described herein may be carried out. The measurements obtained via the method may continue to be gathered and analyzed concurrently over a period of time to calculate ongoing AHIs of the patient. The measurements described herein may then be analyzed in relation to the calculated AHIs of the patient to provide further diagnoses and suggestions for treatment.

In this manner, using the present disclosure is akin to participating in a sleep study each night. This is beneficial for patients since most patients with sleep disorders participate in a sleep study as infrequently as once per year. Said patients then receive one diagnosis until their next sleep study, even if their sleeping behavior changes before their next sleep study. By using the present disclosure, a patient can obtain updated diagnoses in real-time as their sleeping behavior changes. Furthermore, the present disclosure may be used in a patient's own home. When patients participate in sleep studies in a medical professional's office or testing center, they may not feel completely comfortable since they are in an unfamiliar environment. Patients therefore may exhibit abnormal sleeping behavior that is not indicative of the sleeping behavior they normally exhibit. By participating in sleep studies in a patient's own home, the patient is made to feel more comfortable, and thereby exhibit sleeping behavior that they would normally exhibit.

The present disclosure also relates to a non-volatile storage media storing programmable instructions that are configured to execute the following method using the PAP system described herein when executed by a processor:
Using the heart rate monitor to obtain heart rate measurements of a patient;
Using the pulse oximeter to obtain oxygen saturation measurements of the patient;
Using the microphone to obtain sound measurements of the patient;
Using the gyroscope to obtain position measurements of the patient;
Using the pressure sensor to obtain pressure measurements of air within the air passage;
Using the pressure measurements to calculate respiratory air flow of the patient;
Using the ammeter to obtain electrical current measurements of the PAP system;
Using the electrical current measurements or the pressure measurements to detect a mask leak; and
Analyzing the pressure measurements, heart rate measurements, oxygen saturation measurements, sound measurements, position measurements, and electrical current measurements concurrently to determine a sleep diagnosis.

The method executed by the non-volatile storage media storing programmable instructions using the processor may also include:
Using the at least one ECG position sensor to obtain ECG position measurements of the at least one ECG sensor;
Using the ECG position measurements to calculate respiratory effort of the patient; and
Analyzing the respiratory effort of the patient concurrently with the pressure measurements, heart rate measurements, oxygen saturation measurements, sound measurements, position measurements, and electrical current measurements to determine the sleep diagnosis.

The present disclosure also relates to a non-volatile storage media storing programmable instructions that are configured to execute the following method using the PAP system described herein when executed by a processor:
Using the heart rate monitor to obtain heart rate measurements of a patient;
Using the pulse oximeter to obtain oxygen saturation measurements of the patient;
Using the microphone to obtain sound measurements of the patient;
Using the gyroscope to obtain position measurements of the patient;
Using the pressure sensor to obtain pressure measurements of air within the air passage;
Using the pressure measurements to calculate respiratory air flow of the patient;
Using the ammeter to obtain electrical current measurements of the PAP system;
Using the electrical current measurements or the pressure measurements to detect a mask leak;
Analyzing the pressure measurements, heart rate measurements, oxygen saturation measurements, sound measurements, position measurements, and electrical current measurements concurrently over a period of time to calculate an initial patient AHI;
Analyzing the pressure measurements, heart rate measurements, oxygen saturation measurements, sound measurements, position measurements, and electrical current measurements concurrently over a period of time to calculate ongoing patient AHIs; and
Analyzing the pressure measurements, heart rate measurements, oxygen saturation measurements, sound measurements, position measurements, and electrical current measurements concurrently to provide a diagnosis.

The method executed by the non-volatile storage media storing programmable instructions using the processor may also include:
Using the at least one ECG position sensor to obtain ECG position measurements of the at least one ECG sensor;
Using the ECG position measurements to calculate respiratory effort of the patient;
Analyzing the respiratory effort of the patient concurrently with the pressure measurements, heart rate measurements, oxygen saturation measurements, sound measurements, position measurements, and electrical current measurements over a period of time to calculate the initial patient AHI;
Analyzing the respiratory effort of the patient concurrently with the pressure measurements, heart rate measurements, oxygen saturation measurements, sound measurements, position measurements, and electrical current measurements concurrently over a period of time to calculate ongoing patient AHIs; and

Analyzing the respiratory effort of the patient concurrently with the pressure measurements, heart rate measurements, oxygen saturation measurements, sound measurements, position measurements, and electrical current measurements concurrently to provide a diagnosis.

The term "diagnosis" (plural "diagnoses") as used herein may be used to describe the identification of the nature of an illness or other problem by examination and/or analysis of symptoms and/or measurements. The term "diagnosis" (plural "diagnoses") as used herein may also be used to describe a suggestion for treatment for said illness or other problem. The PAP system may provide diagnoses to a patient via notifications on patient's device or via notifications on a user interface of the PAP unit of the PAP system. The PAP system may provide diagnoses to a medical professional via notifications on a medical professional's device.

### Brief Description of the Figures

Figure 1 is a diagram showing the transfer of data between sensors, a processor, a patient's device, and a medical professional's device of a PAP system via a transmitter of the PAP system.
Figure 2 is a side view of a mask and PAP unit of a PAP system. The PAP unit is shown in a cross-section view.
Figure 3 is a side view of a patient wearing a mask and wearable articles of a PAP system while sleeping.
Figure 4 is a flowchart showing a method of providing a diagnosis using a PAP system.
Figure 5 is a flowchart showing a method of performing at least one diagnosis using a PAP system.
Figure 6 is a flowchart showing a method of providing at least one diagnosis using a PAP system.
Figure 7 is a flowchart showing a method of providing at least one diagnosis using a PAP system.
Figure 8 is a flowchart showing a method of providing at least one diagnosis using a PAP system.
Figure 9 is a flowchart showing a method of providing a diagnosis using a PAP system.
Figure 10 is a flowchart showing a method of providing at least one diagnosis using a PAP system.
Figure 11 is a flowchart showing a method of providing at least one diagnosis using a PAP system.
Figure 12 is a flowchart showing a method for performing a polysomnography using a PAP system.
Figure 13 is a flowchart showing a method for performing a polysomnography using a PAP system.
Figure 14 is a flowchart showing a method for performing a polysomnography using a PAP system.
Figure 15 is a flowchart showing a method for performing a polysomnography using a PAP system.
Figure 16 is a flowchart showing a method for performing a polysomnography using a PAP system.

### Detailed Description

The description provided herein describes example embodiments of the present invention and is not intended to limit the invention to any particular embodiment, component, feature, use, design, step(s), order of operations, function, or any other property. Furthermore, the drawings provided herein show example embodiments of the present invention and are not intended to limit the invention to any particular embodiment, component, feature, use, design, step(s), order of operations, function, or any other property.

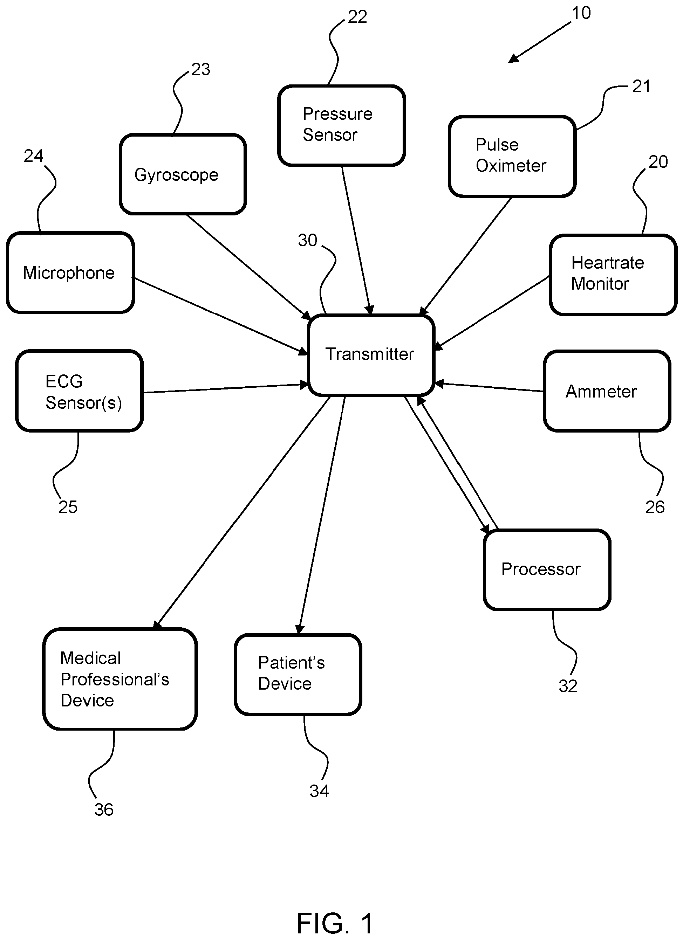

As shown in FIG. 1, a PAP system 10 may have multiple sensors such as a heart rate monitor 20, a pulse oximeter 21, a pressure sensor 22, a gyroscope 23, a microphone 24, at least one ECG sensor 25, and an ammeter 26. The sensors obtain measurement data from a patient and from the PAP system 10 and transmit the measurement data in the form of non-transitory computer-readable media to a processor 32 via a transmitter 30. The sensors may also transfer the measurement data directly to a patient's device 34 and/or a medical professional's device 36 via the transmitter 30. The processor 32 analyzes the measurement data and sends its analyses in the form of non-transitory computer-readable media to the patient's device 34 and/or medical professional's device 36 via the transmitter 30.

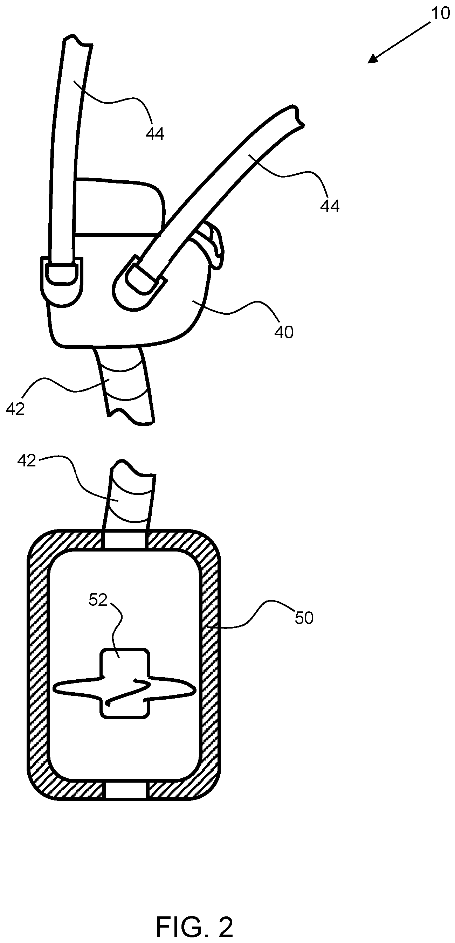

As shown in FIG. 2, a blower fan 52 is housed within a PAP unit 50 of a PAP system 10. The blower fan 52 takes ambient air and sends the air to a mask 40 via an air passage 42. As shown in FIG. 2, the air passage 42 is a hose that connects the PAP unit 50 and the mask 40. Two straps 44 are configured on the mask 40 and may be used to secure the mask 40 to a patient's head. The processor and transmitter of the PAP system 10 may be configured within the PAP unit 50.

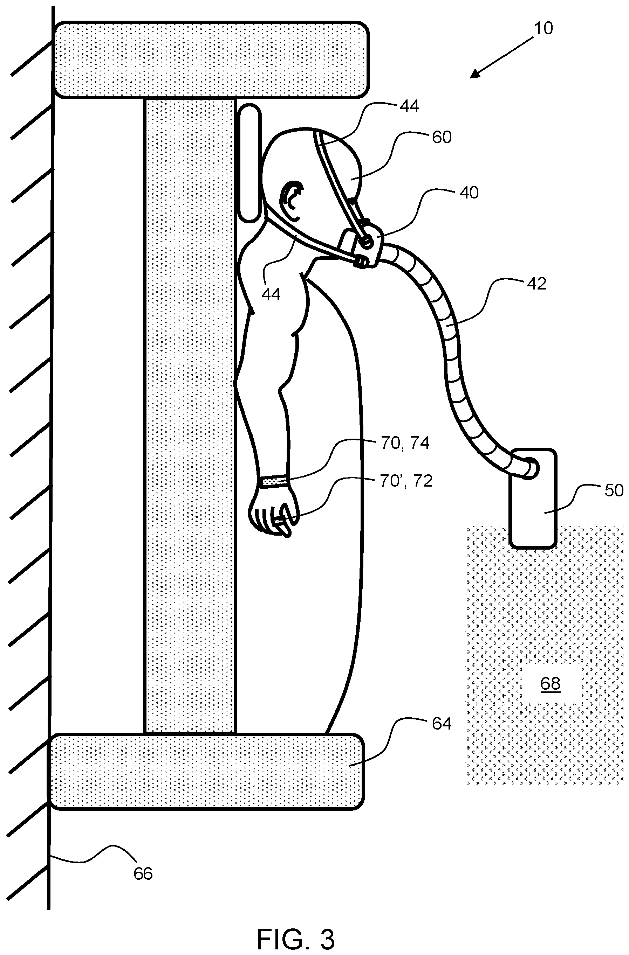

As shown in FIG. 3, the patient 60 may use the PAP system 10 while sleeping. FIG. 3 is oriented whereby a patient's bed 64 is oriented in a vertical direction in relation to the page in order to maximize drawing space on the page of FIG. 3. Therefore, the ground 66 is shown in FIG. 3 to illustrate a horizontal sleeping position of the patient 60. The mask 40 of the PAP system 10 is secured to the patient's head by the straps 44. The mask 40 is connected to the PAP unit 50 by the air passage 42, which is shown as a hose in FIG. 3. The air passage 42 is configured between the PAP unit 50 and the patient's face. The PAP unit 50 is mounted on a wall 68. The PAP unit 50 may be mounted on the wall 68 to prevent the air passage 42 from obstructing the patient's movement while the patient 60 sleeps. The PAP unit 50 may also be mounted on the wall 68 to prevent the patient 60 from obstructing the air flowing through the air passage 42 if the patient 60 moves while sleeping.

Also as shown in FIG. 3, the patient 60 wears a wearable article 70, 70'. The wearable article 70, 70' may be but is not limited to a ring 72 worn around the patient's finger, and/or a bracelet 74 worn around the patient's wrist. Some of the sensors of the PAP system 10 may be configured on or within the wearable article 70, 70'. Other sensors of the PAP system 10 may be configured on or within the mask 40. Other sensors of the PAP system 10 may be configured within the air passage 42. Other sensors of the PAP system may be configured within the PAP unit 50.

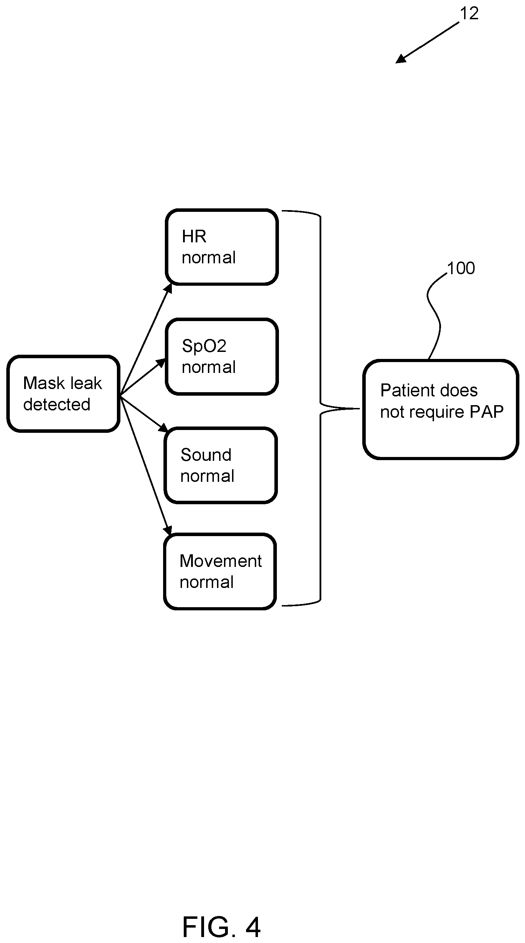

As shown in FIG. 4, the PAP system detects a mask leak as part of a method for providing a diagnosis 12. The system obtains heart rate measurements (HR), oxygen saturation measurements (SpO2), sound measurements, and position (movement) measurements. The system determines that the heart rate measurements are within a normal range, the oxygen saturation measurements are within a normal range, the sound measurements are within a normal range, and the movement measurements are within a normal range. Due to these measurements being analyzed concurrently, the diagnosis 100 that is provided is that the patient does not require PAP therapy. If the heart rate, oxygen saturation, and movement measurements were not obtained, the system would be relying mostly on the detection of a mask leak, and may assume that the patient does require PAP therapy and possibly suffers from positional OSA.

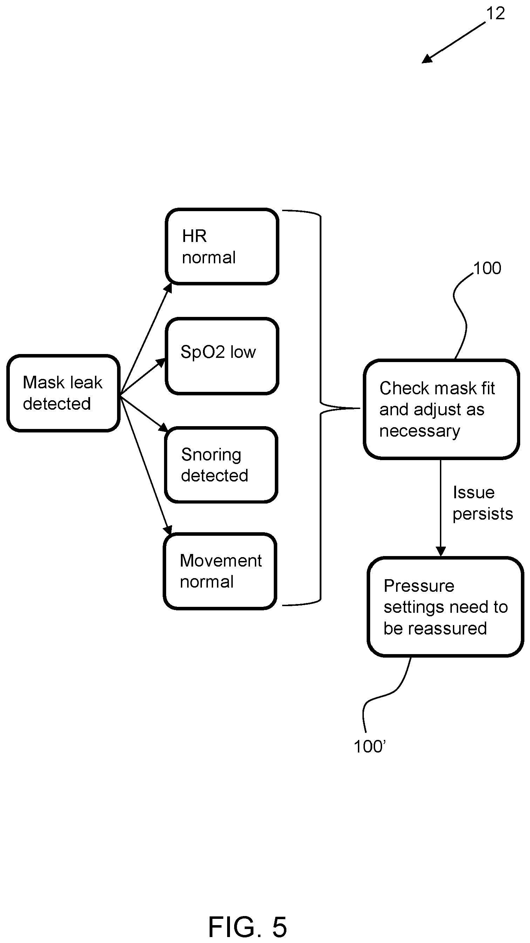

As shown in FIG. 5, the PAP system detects a mask leak as part of a method for providing a diagnosis 12. The system obtains heart rate measurements (HR), oxygen saturation measurements (SpO2), sound measurements, and movement measurements. The system determines that the heart rate measurements are within a normal range and the movement measurements are within a normal range. However, the system also determines that the sound measurements are not within a normal range, and therefore detects snoring from the patient. The system also determines that the oxygen saturation measurements are low (below a normal range).

Due to these measurements being analyzed concurrently, the diagnosis 100 that is provided is to check the fit of the mask against the patient's face and adjust if necessary. In this scenario, the system was not 100% confident in the initial detection of the mask leak before analyzing the oxygen saturation measurements and sound measurements. However, the sound measurements led to the detection of snoring by the PAP system, and snoring is known in the field of sleep therapy to lead to a drop in oxygen saturation levels. Therefore, since the PAP system analyzed the oxygen saturation measurements and found them to be low, the presence of a mask leak is confirmed, and therefore the diagnosis 100 is to check the fit of the mask and adjust as necessary. If the oxygen saturation measurements and sound measurements had not been obtained and analyzed concurrently with the other measurements, then the initial detection of the mask leak would have been assumed to be an error since the heart rate measurements and movement measurements are within a normal range.

Also as shown in FIG. 5, if the issue persists (i.e. if the oxygen saturation measurements and sound measurements continue to be outside of a normal range after checking and/or adjusting the fit of the mask), then a secondary diagnosis 100' is to reassure the pressure settings of the PAP system to ensure that the patient is receiving adequate air flow to treat their sleep apnea.

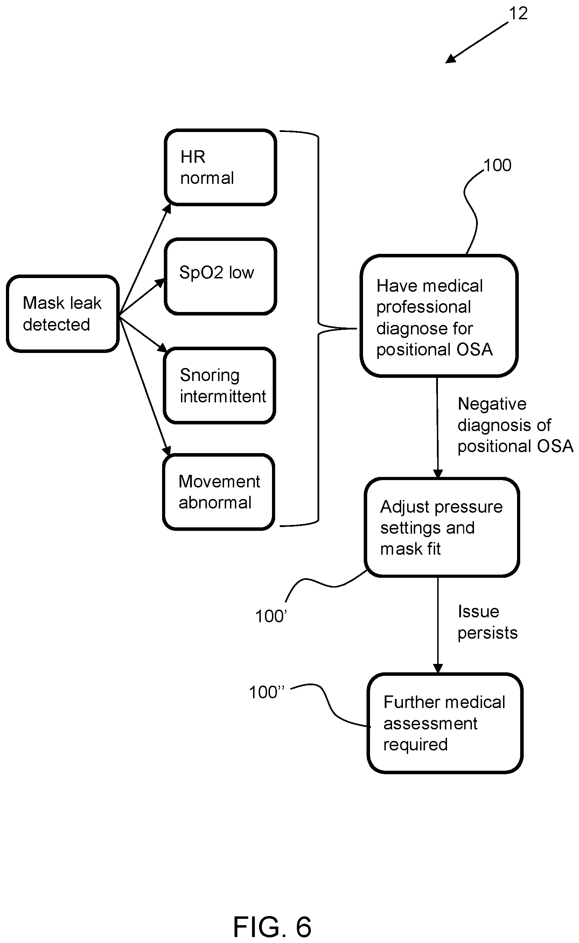

As shown in FIG. 6, the PAP system detects a mask leak as part of a method for providing a diagnosis 12. The system obtains heart rate measurements (HR), oxygen saturation measurements (SpO2), sound measurements, and movement measurements. The system determines that the heart rate measurements are within a normal range. However, the system also detects snoring at times (snoring intermittent), determines that the movement measurements are abnormal (not within a normal range), and determines that the oxygen saturation measurements are low. Due to these measurements being analyzed concurrently, the diagnosis 100 that is provided is to have a medical professional diagnose the patient for positional obstructive sleep apnea. Since the snoring is intermittent, it can be determined that the snoring is caused by the patient moving in their sleep (movement abnormal). Since the patient's oxygen saturation measurements are low, this snoring due to movement can be attributed to positional OSA. The key to this diagnosis is that the movement measurements were analyzed in concurrence with the sound measurements and oxygen saturation measurements. If the movement measurements were not obtained and analyzed, the system may have diagnosed a different disorder rather than possible positional OSA.

Also as shown in FIG. 6, the medical professional provides a negative diagnosis of positional OSA. The medical professional may provide a diagnosis directly to the patient. Alternatively, the medical professional may provide a diagnosis to the PAP system via a medical professional's device in the form of non-transitory computer-readable media. In this scenario, the PAP system provides a secondary diagnosis 100' of adjusting the pressing settings and the fit of the mask. Since positional OSA has been ruled out, this leads the system to believe that the abnormal sound and oxygen saturation measurements are due to the mask leak. If these abnormal measurements persist after adjusting the pressure settings and/or mask fit, the system provides a tertiary diagnosis 100" of suggesting a further medical assessment, such as a more robust polysomnography.

The system may determine if the abnormal measurements persist by continuous gathering and analyzing of the measurements described herein. The system may perform new analyses and provide new diagnoses after a period of time following a previous analysis or diagnosis in order to allow for any adjustments to take effect on the patient's sleep. This period of time may be 24 hours or more, 48 hours or more, 72 hours or more, or any range between and including the values provided. During this period of time, new analyses/diagnoses may only be provided if the patient uses the PAP system while sleeping for a minimum sleep period. This minimum sleep period may be 4 hours or more, 6 hours or more, 8 hours or more, or any range between and including the values provided.

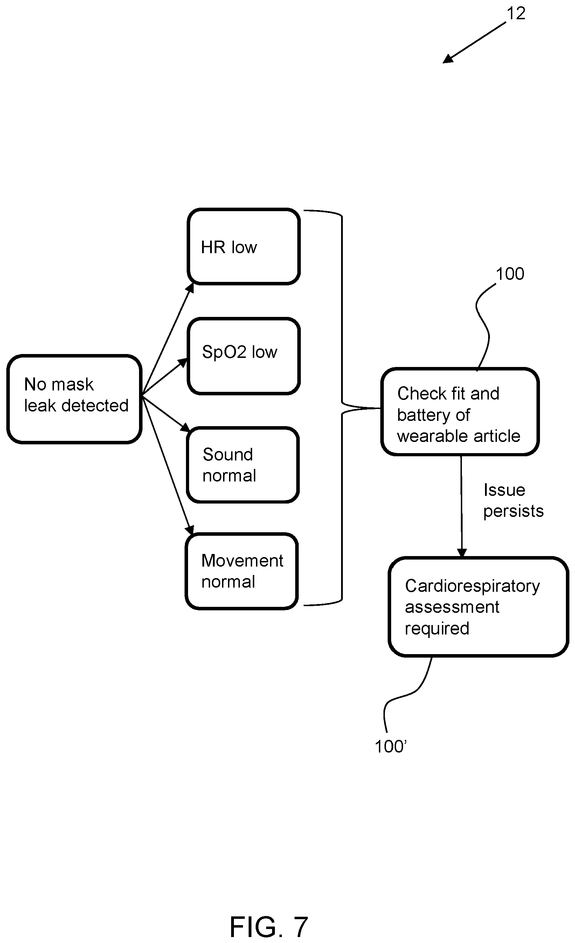

As shown in FIG. 7, the PAP system does not detect a mask leak as part of a method for providing a diagnosis 12. The system obtains heart rate measurements (HR), oxygen saturation measurements (SpO2), sound measurements, and movement measurements. The system determines that the heart rate and oxygen saturation measurements are low. The system also determines that the sound and movement measurements are within a normal range. Due to these measurements being analyzed concurrently, the diagnosis 100 that is provided is to check the fit of the wearable article on the patient, as well as the battery of the wearable article. In this scenario, the heart rate and oxygen saturation measurements are obtained from sensors configured on or within the wearable article. The sound and movement measurements may be obtained from sensors configured on or within the mask. Alternatively, the sound and movement measurements may be obtained from sensors configured on or within the PAP unit and/or wearable article. Therefore, when the sound and movement measurements are within a normal range and the heart rate and oxygen saturation measurements are below a normal range with no mask leak being detected, it can be determined that there is an issue with the sensors that obtain the heart rate and oxygen saturation measurements, and therefore an issue with the wearable article. The wearable article may be troubleshooted by cleaning the wearable article, by replacing the battery used to power the wearable article, and/or by replacing the various sensors configured on or within the wearable article.

Also as shown in FIG. 7, if the heart rate and oxygen saturation measurements are still low after troubleshooting the wearable article, the system provides a secondary diagnosis 100' of suggesting a cardiorespiratory assessment to assess the patient's low heart rate and oxygen saturation without other indicators (mask leak, snoring, and movement) of sleep apnea.

The system may determine if the heart rate and oxygen saturation measurements are still low by continuous gathering and analyzing of the measurements described herein. The system may perform new analyses and provide new diagnoses after a period of time following a previous analysis or diagnosis in order to allow for any adjustments to take effect on the patient's sleep. This period of time may be 24 hours or more, 48 hours or more, 72 hours or more, or any range between and including the values provided. During this period of time, new analyses/diagnoses may only be provided if the patient uses the PAP system while sleeping for a minimum sleep period. This minimum sleep period may be 4 hours or more, 6 hours or more, 8 hours or more, or any range between and including the values provided.

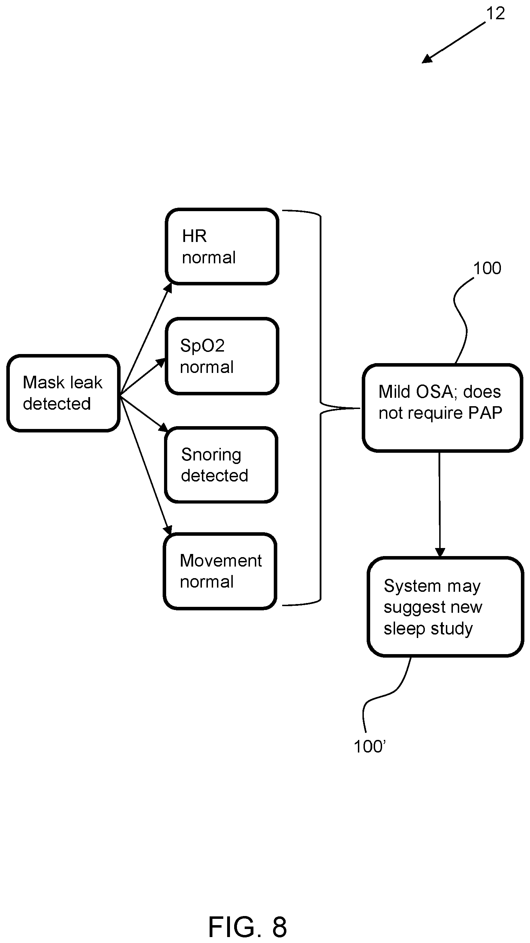

As shown in FIG. 8, the PAP system detects a mask leak as part of a method for providing a diagnosis 12. The system obtains heart rate measurements (HR), oxygen saturation measurements (SpO2), sound measurements, and movement measurements. The system determines that the heart rate measurements, oxygen saturation measurements, and movement measurements are within a normal range. However, the system also detects snoring. Due to these measurements being analyzed concurrently, the diagnosis 100 that is provided is that the patient suffers from mild OSA and does not require PAP therapy. The system also provides a secondary diagnosis 100' of suggesting a new sleep study. If the system had not analyzed these measurements concurrently, it may have provided a diagnosis of using PAP therapy unnecessarily, since it would not have taken into account that the heart rate measurements and oxygen saturation measurements were normal, even though snoring was present.

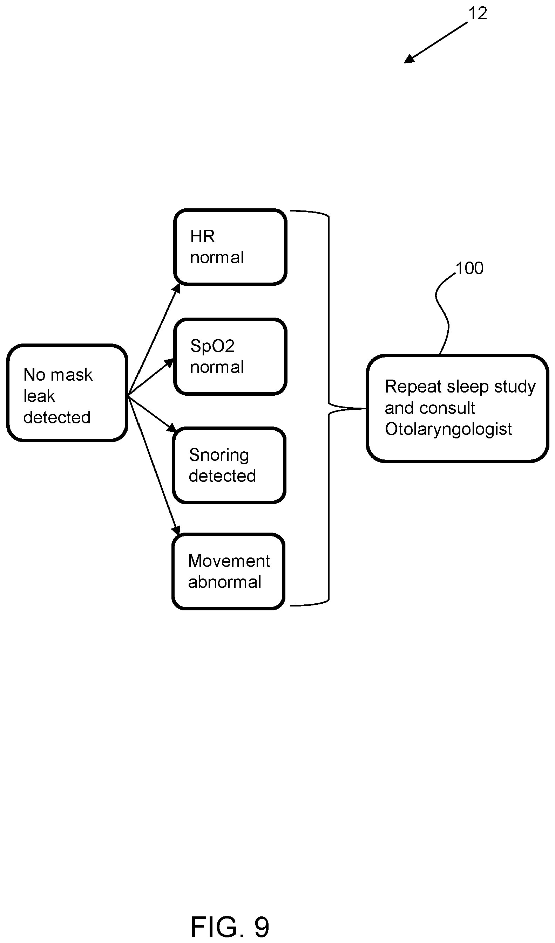

As shown in FIG. 9, the PAP system does not detect a mask leak as part of a method for providing a diagnosis 12. The system obtains heart rate measurements (HR), oxygen saturation measurements (SpO2), sound measurements, and movement measurements. The system determines that the heart rate measurements and oxygen saturation measurements are within a normal range. However, the system also detects snoring, and determines that the movement measurements are abnormal. Due to these measurements being analyzed concurrently, the diagnosis 100 that is provided is that the patient should repeat the sleep study carried out per the gathering and analysis of these measurements, as well as to consult an otolaryngologist. Since there is no mask leak, the heart rate measurements are within a normal range, and the oxygen saturation measurements are within a normal range, it is likely that the patient does not suffer from sleep apnea. The presence of snoring leads the system to recommend consultation with an otolaryngologist. The system further suggests to repeat the sleep study (continue gathering and analyzing the heart rate measurements, oxygen saturation measurements, sound measurements, and movement measurements) to ensure that the patient does not suffer from sleep apnea.

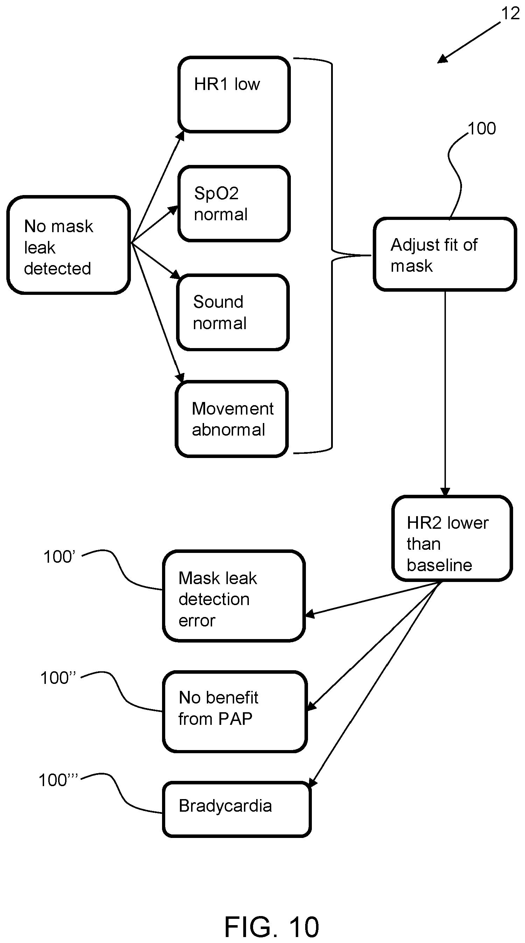

As shown in FIG. 10, the PAP system does not detect a mask leak as part of a method for providing a diagnosis 12. The system obtains initial heart rate measurements (HR1), oxygen saturation measurements (SpO2), sound measurements, and movement measurements. The system determines that the initial heart rate measurements are low, the oxygen saturation measurements are within a normal range, the sound measurements are within a normal range, and the movement measurements are not within a normal range. Due to these measurements being analyzed concurrently, the diagnosis 100 that is provided is to adjust the fit of the mask against the patient's face. Low heart rate concurrent with movement during sleep are generally indicative of sleep apnea. However, the lack of a mask leak and the lack of snoring leads to the conclusion that other signals of sleep apnea, such as snoring, are not being detected, and therefore the fit of the mask should be adjusted to attempt to correct the patient's heart rate or detect snoring.

Also as shown in FIG. 10, the system obtains further heart rate measurements (HR2) and compares the further heart rate measurements against a "baseline" of the initial heart rate measurements (HR1). If the further heart rate measurements are lower than the baseline, the system provides a secondary diagnosis 100' of an error in detecting a mask leak. A mask leak detection error can be due to a technical/electronic problem associated with any of the sensors such as the ammeter, pressure sensor, microphone, gyroscope, heart rate monitor, pulse oximeter, at least one ECG sensor, and/or at least one ECG position sensor. If the system detects an error in mask leak detection, the system may recommend that the patient replace one or more of the sensors, or replace the system entirely.

The further heart rate measurements may be analyzed and the secondary diagnosis 100' provided after a period of time following the first diagnosis 100 in order to allow for any adjustments to take effect on the patient's sleep. This period of time may be 24 hours or more, 48 hours or more, 72 hours or more, or any range between and including the values provided. During this period of time, the further heart rate measurements may only be analyzed and the secondary diagnosis 100' only provided if the patient uses the PAP system while sleeping for a minimum sleep period. This minimum sleep period may be 4 hours or more, 6 hours or more, 8 hours or more, or any range between and including the values provided.

The system also provides a tertiary diagnosis 100'' of suggesting the patient is not benefiting from PAP therapy. In such scenarios, a repeat PSG is recommended to diagnose the severity of the patient's OSA. A patient not benefiting from PAP therapy may require corrective surgery to treat their OSA. The system also provides a fourth diagnosis 100‴ of bradycardia, or low heart rate. Bradycardia may not be related to OSA and needs to be assessed independently by a medical professional. The secondary diagnosis 100', tertiary diagnosis 100'', and fourth diagnosis 100‴ are determined due to the fact that the further heart rate measurements are still low without the presence of snoring or other indicators of sleep apnea, even after the fit of the mask was adjusted.

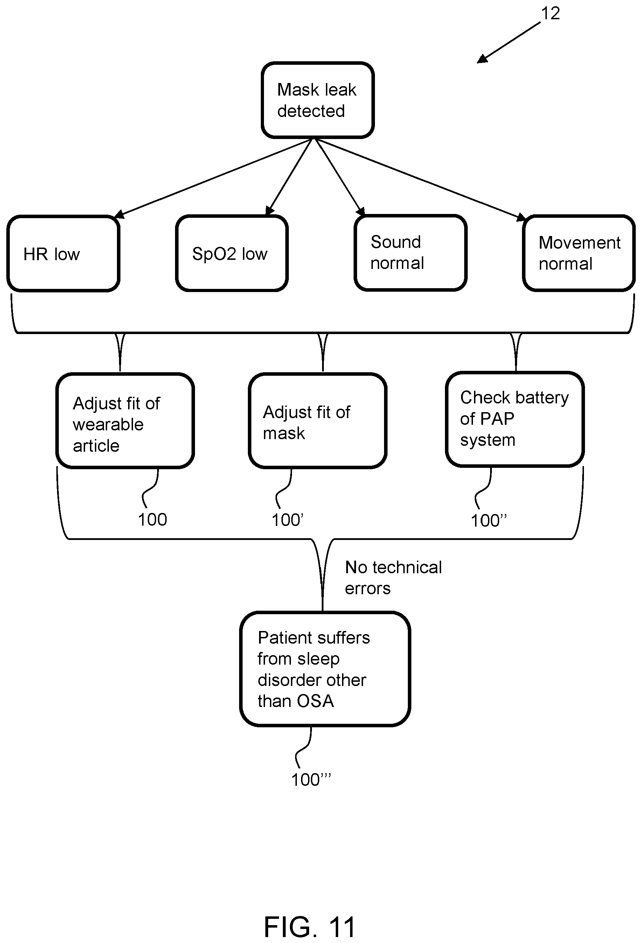

As shown in FIG. 11, the PAP system detects a mask leak as part of a method for providing a diagnosis 12. The system obtains heart rate measurements (HR), oxygen saturation measurements (SpO2), sound measurements, and movement measurements. The system determines that the heart rate measurements are low, the oxygen saturation measurements are low, the sound measurements are within a normal range, and the movement measurements are within a normal range. Due to these measurements being analyzed concurrently, the system gives a first diagnosis 100 of suggesting adjusting the fit of the wearable article. The system also gives a secondary diagnosis 100' of suggesting adjusting the fit of the mask against the patient's face. The system also gives a tertiary diagnosis 100'' of suggesting checking the battery of the PAP system, which may be configured within the mask, the wearable article, the PAP unit, or any combination of the three. The heart rate measurements and oxygen saturation measurements being low, together with a mask leak, are indicative of improper use of the PAP system. Since the movement measurements are within a normal range, this leads to the conclusion that instead of improper use of the PAP system, there is a technical error, such as the fit of the wearable article, the fit of the mask, or the battery of the mask and/or wearable article.

Also as shown in FIG. 11, if no technical errors are present in the PAP system, then the system provides a fourth diagnosis 100‴ of a sleep disorder other than OSA, since snoring would be present if the patient suffered from OSA.

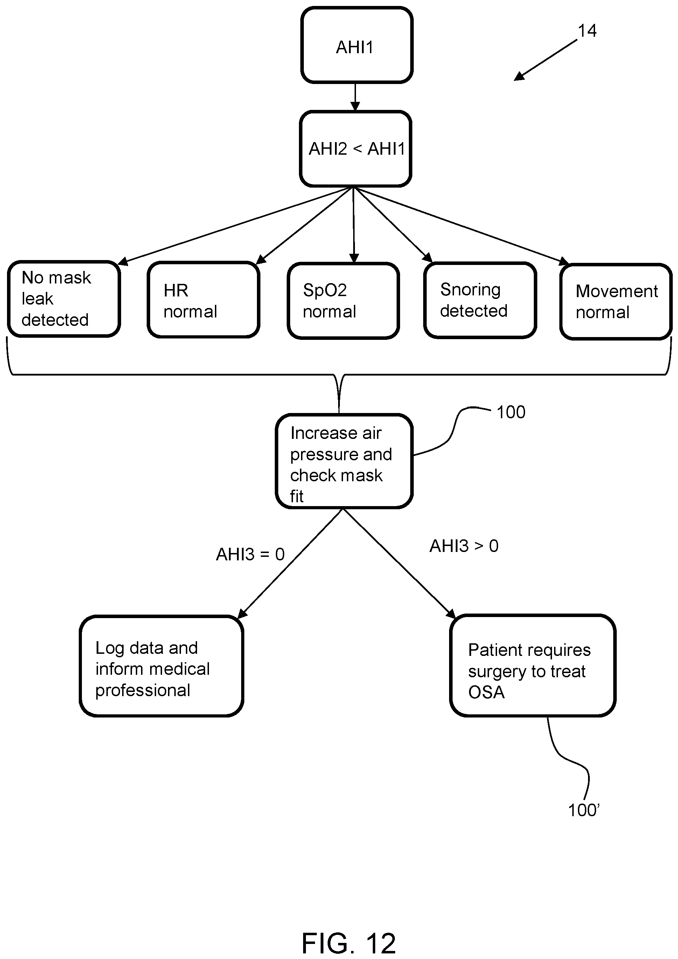

As shown in FIG. 12, the PAP system calculates an initial apnea-hypopnea index (AHI1) as part of a method for performing a polysomnography 14. AHI1 is calculated by continual gathering and analysis of heart rate measurements (HR) of a patient, oxygen saturation measurements (SpO2) of the patient, sound measurements of the patient, position (movement) measurements of the mask of the PAP system, electrical activity measurements of the patient's heart, ECG position measurements of at least one ECG sensor, pressure measurements of the air within the air passage of the mask of the PAP system, and electrical current measurements of the PAP system. The system continues gathering and monitoring these measurements after calculating AHI1 to then calculate a second apnea-hypopnea index (AHI2).

As shown in FIG. 12, AHI2 is calculated to be less than AHI1. No mask leak is detected. The heart rate measurements, oxygen saturation measurements, and movement measurements are within a normal range. The sound measurements are outside of a normal range whereby the system detects snoring. The system provides a diagnosis 100 of suggesting increasing the air pressure of the mask and to check the fit of the mask. The system continues to gather and analyze the measurements described herein to calculate a third apnea-hypopnea index (AHI3).

As shown in FIG. 12, if AHI3 = 0, the system logs the data from the various measurements and transmits this data to a medical professional. If AHI3 is greater than 0, the system provides a secondary diagnosis 100' of surgery being required to treat the patient's OSA. In this manner, the system performs as "split study" polysomnography for diagnoses.

AHI2 may be calculated after a period of time following the calculation of AHI1. AHI3 may be calculated after a period of time following the calculation of AHI2. This period of time may allow for any adjustments to take effect on the patient's sleep. This period of time may be 24 hours or more, 48 hours or more, 72 hours or more, or any range between and including the values provided. During this period of time, AHI2/AHI3 may only be calculated if the patient uses the PAP system while sleeping for a minimum sleep period. This minimum sleep period may be 4 hours or more, 6 hours or more, 8 hours or more, or any range between and including the values provided.

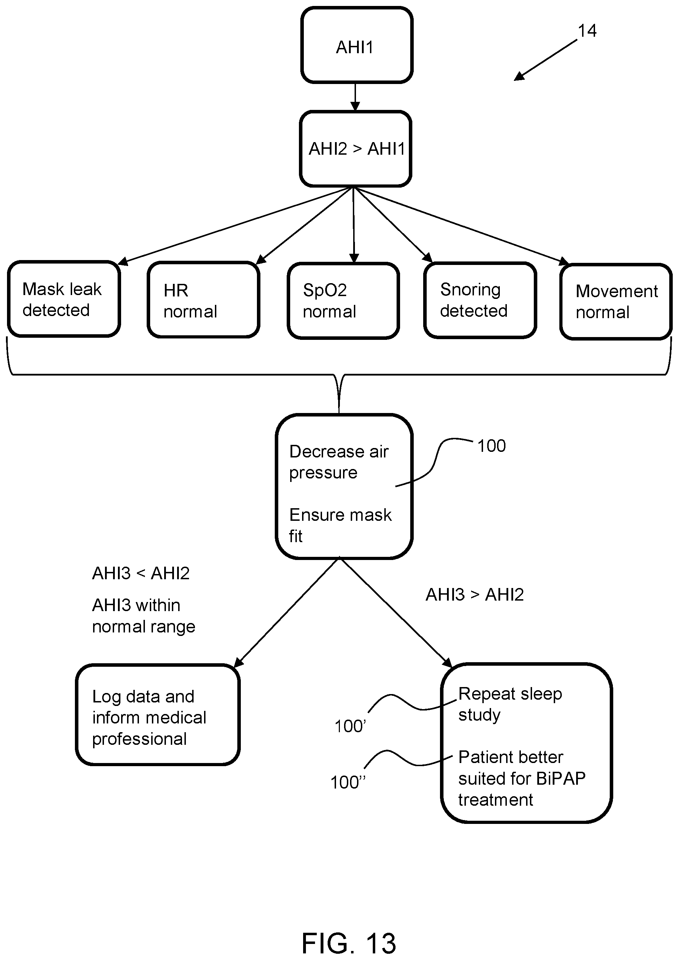

As shown in FIG. 13, the PAP system calculates an initial apnea-hypopnea index (AHI1) as part of a method for performing a polysomnography 14. AHI1 is calculated as a baseline AHI to use for comparison during the polysomnography 14. As the patient continues to use the system, the system continues gathering and analyzing the measurements described herein to then calculate a second apnea-hypopnea index (AHI2). As shown in FIG. 13, AHI2 is calculated to be greater than AHI1, which indicates an issue with the system or with the patient's overall sleep treatment. After calculating AHI2, a mask leak is detected. The heart rate measurements, oxygen saturation measurements, and movement measurements are within a normal range. The sound measurements are outside of a normal range whereby the system detects snoring. As preliminary troubleshooting, the system provides a diagnosis 100 of both suggesting decreasing the air pressure of the mask and ensuring the fit of the mask. After these adjustments are made, the system continues to gather and analyze the measurements described herein to calculate a third apnea-hypopnea index (AHI3).

As shown in FIG. 13, if AHI3 is less than AHI2 and if AHI3 is within a normal range, it can be concurred that the preliminary troubleshooting was effective in improving the patient's vital signs. A normal range for an AHI may be 5 or less, 7 or less, 10 or less, or any range between and including the values provided. If AHI3 is less than AHI2 and if AHI3 is within a normal range, the system logs the data from the various measurements and transmits this data to a medical professional. If AHI3 is greater than AHI2, the system provides a secondary diagnosis 100' of repeating the polysomnography 14. The system also provides a tertiary diagnosis 100" of suggesting BiPAP therapy rather than CPAP therapy. The tertiary diagnosis 100" is provided if the PAP system that the patient is using is a CPAP system.

AHI2 may be calculated after a period of time following the calculation of AHI1. AHI3 may be calculated after a period of time following the calculation of AHI2. This period of time may allow for any adjustments to take effect on the patient's sleep. This period of time may be 24 hours or more, 48 hours or more, 72 hours or more, or any range between and including the values provided. During this period of time, AHI2/AHI3 may only be calculated if the patient uses the PAP system while sleeping for a minimum sleep period. This minimum sleep period may be 4 hours or more, 6 hours or more, 8 hours or more, or any range between and including the values provided.

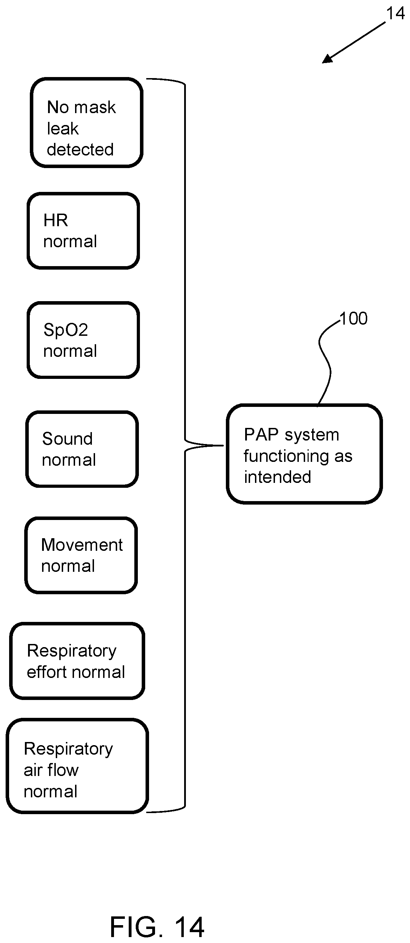

As shown in FIG. 14, the PAP system performs an ongoing polysomnography 14 by continuously gathering and analyzing heart rate measurements (HR) of a patient, oxygen saturation measurements (SpO2) of the patient, sound measurements of the patient, position (movement) measurements of the mask of the PAP system, electrical activity measurements of the patient's heart (or alternatively ECG position measurements of at least one ECG sensor), pressure measurements of the air within the air passage of the mask of the PAP system, and electrical current measurements of the PAP system. A processor of the PAP system uses the pressure measurements to calculate respiratory air flow of the patient. The processor uses the electrical activity measurements of the patient's heart (or alternatively ECG position measurements of at least one ECG sensor) to calculate respiratory effort of the patient. The processor uses the electrical current measurements of the PAP system (or alternatively the pressure measurements) to monitor for a mask leak.

As shown in FIG. 14, no mask leak is detected. The heart rate measurements, oxygen saturation measurements, sound measurements, and movement measurements are all within a normal range. The respiratory effort of the patient and respiratory air flow of the patient are calculated to be within a normal range. Therefore, the system provides a diagnosis 100 that the PAP system is functioning as intended. The PAP system continues to gather and analyze the measurements described herein as part of the polysomnography 14 in case any of the measurements or calculations described herein stray from within a normal range, whereby the PAP system would provide another diagnosis.

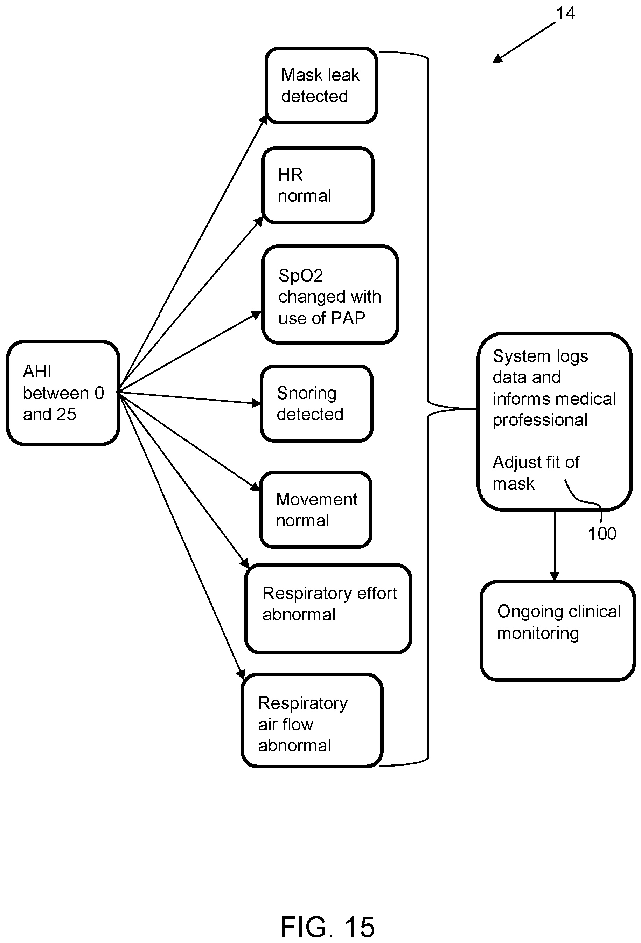

As shown in FIG. 15, the PAP system calculates an apnea-hypopnea index (AHI) as part of a method for performing a polysomnography 14. AHI is calculated by continual gathering and analysis of the various measurements described herein. The AHI in FIG. 15 is calculated to be between 0 and 25. The system detects a mask leak, as well as snoring. The heart rate measurements and movement measurements are within a normal range. The patient's respiratory effort and respiratory air flow are calculated to be outside of a normal range. The oxygen saturation measurements are detected to have changed over time with the use of the PAP system. The system logs the data from the various measurements and transmits this data to a medical professional. The system provides a diagnosis 100 of suggesting adjusting the fit of the mask due to the detection of the mask leak, detection of snoring, detection of the change in oxygen saturation measurements, and calculation of abnormal respiratory effort and respiratory air flow. The system does not provide further diagnoses of more severe conditions or more extensive treatment at this time due to the heart rate measurements being within a normal range and the movement measurements being within a normal range. However, the system does continue to gather and analyze the measurements described herein in case any of the measurements change and warrant a new diagnosis.

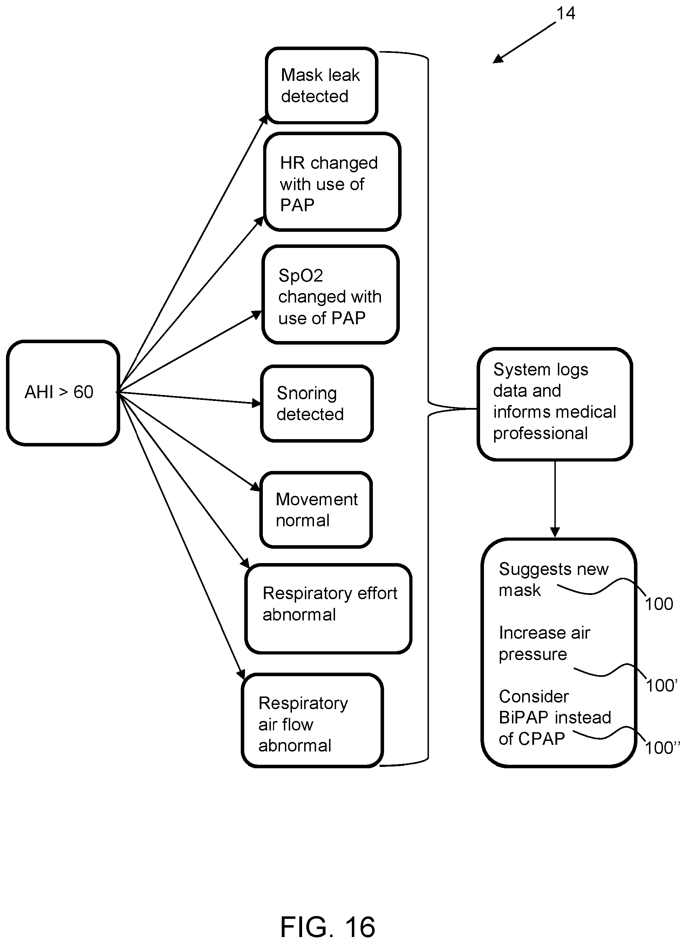

As shown in FIG. 16, the PAP system calculates an apnea-hypopnea index (AHI) as part of a method for performing a polysomnography 14. AHI is calculated by continual gathering and analysis of the various measurements described herein. The AHI in FIG. 16 is calculated to be between greater than 60, which may be indicative of severe OSA. The system detects a mask leak and snoring. The movement measurements are within a normal range. The heart rate measurements and oxygen saturation measurements are calculated to have changed over time with use of the PAP system. The patient's respiratory effort and respiratory air flow are calculated to be outside of a normal range.

The system logs the data from the various measurements and transmits this data to a medical professional. The system provides a first diagnosis 100 of suggesting a new mask, and a second diagnosis 100' of increasing the air pressure of the mask. The first diagnosis 100 and second diagnosis 100' are made as preliminary measures since a mask leak is detected. A tertiary diagnosis 100" is also provided to consider BiPAP as a preferred PAP method for the patient instead of CPAP. The tertiary diagnosis 100'' is only provided if the PAP system currently being used by the patient is a CPAP system. The tertiary diagnosis 100" is made due to the fact that the patient is using the CPAP system but is still demonstrating severe OSA due to their high AHI along with changes in heart rate measurements and oxygen saturation measurements, and along with abnormal respiratory effort and respiratory air flow. Therefore, the system determines that CPAP may not be the best therapy for the patient in treating their OSA.

## Claims

1. A PAP system (10) comprising:
a PAP unit (50) comprising:
a blower fan (52);
an air passage (42) configured between the PAP unit and the face of a patient;
a mask (40);
a processor (32);
a heart rate monitor (20), wherein the heart rate monitor obtains heart rate measurements of the patient;
a pulse oximeter (21), wherein the pulse oximeter obtains oxygen saturation measurements of the patient;
a microphone (24), wherein the microphone obtains sound measurements of the patient;
a gyroscope (23), wherein the gyroscope obtains position measurements of the patient;
a pressure sensor (22), wherein the pressure sensor obtains pressure measurements of air within the air passage, wherein the processor uses said pressure measurements to calculate respiratory air flow of the patient; and
an ammeter (26), wherein the ammeter obtains electrical current measurements of the PAP system, wherein the processor uses said electrical current measurements to detect a mask leak,
wherein the pressure measurements, heart rate measurements, oxygen saturation measurements, sound measurements, position measurements, and electrical current measurements are analyzed concurrently by the processor to determine a sleep diagnosis
wherein:
a) a mask leak is detected,
and wherein the heart rate measurements are within a normal range,
and wherein the oxygen saturation measurements are within a normal range,
and wherein the sound measurements are within a normal range,
and wherein the position measurements are within a normal range,
whereby the diagnosis that is determined by the PAP system is that the patient does not require PAP treatment; and/or
b) a mask leak is detected,
wherein the heart rate measurements are within a normal range,
and wherein the oxygen saturation measurements are not within a normal range,
and wherein the sound measurements are not within a normal range,
and wherein the position measurements are within a normal range,
whereby the diagnosis that is determined by the PAP system is that a determination needs to be made as to whether the mask fits securely on the patient's face; and/or
c) wherein no mask leak is detected,
and wherein the heart rate measurements are not within a normal range,
and wherein the oxygen saturation measurements are within a normal range,
and wherein the sound measurements are within a normal range,
and wherein the position measurements are not within a normal range,
whereby the diagnosis that is determined by the PAP system is that the mask needs to be re-fitted to the patient;.

2. The PAP system of claim 1, wherein the processor uses the pressure measurements to detect the mask leak.

3. The PAP system of claim 1, further comprising:
at least one ECG sensor (25); and
at least one ECG position sensor configured on each of the at least one ECG sensor,
wherein the at least one ECG position sensor obtains ECG position measurements of the at least one ECG sensor,
wherein the processor uses the ECG position measurements to calculate respiratory effort of the patient,
and wherein the respiratory effort is analyzed concurrently with the pressure measurements, heart rate measurements, oxygen saturation measurements, sound measurements, position measurements, and electrical current measurements to determine the sleep diagnosis.

4. The PAP system of claim 1, wherein a mask leak is detected,
and wherein the heart rate measurements are within a normal range,
and wherein the oxygen saturation measurements are not within a normal range,
and wherein the sound measurements are within a normal range,
and wherein the position measurements are not within a normal range,
whereby the diagnosis that is determined by the PAP system is that patient suffers from positional obstructive sleep apnea; and/or
the PAP system of claim 1, wherein no mask leak is detected,
and wherein the heart rate measurements are not within a normal range,
and wherein the oxygen saturation measurements are not within a normal range,
and wherein the sound measurements are within a normal range,
and wherein the position measurements are within a normal range,
whereby the diagnosis that is determined by the PAP system is that the heart rate monitor and pulse oximeter are not functioning as intended.

5. The PAP system of claim 1, wherein a mask leak is detected,
and wherein the heart rate measurements are within a normal range,
and wherein the oxygen saturation measurements are within a normal range,
and wherein the sound measurements are not within a normal range,
and wherein the position measurements are within a normal range,
whereby the diagnosis that is determined by the PAP system is that the patient suffers from mild obstructive sleep apnea and does not require PAP treatment; and/or the PAP system of claim 1, wherein no mask leak is detected,
and wherein the heart rate measurements are within a normal range,
and wherein the oxygen saturation measurements are within a normal range,
and wherein the sound measurements are not within a normal range,
and wherein the position measurements are not within a normal range,
whereby the diagnosis that is determined by the PAP system is that the patient requires further sleep studies.

6. The PAP system of claim 1, wherein the gyroscope is configured on a wearable article (70, 70'),
and wherein the pressure sensor is configured within the air passage,
and wherein the processor and the ammeter are configured within the PAP unit;
and/or The PAP system of claim 1, wherein the heart rate monitor and the pulse oximeter are configured on a wearable article worn by the patient.

7. A non-volatile storage media storing programmable instructions that are configured to execute a method using the PAP system according to any of the preceding claims when executed by the processor for performing a diagnosis using the PAP system of claim 1, the method comprising:
using the heart rate monitor to obtain heart rate measurements of a patient;
using the pulse oximeter to obtain oxygen saturation measurements of the patient;
using the microphone to obtain sound measurements of the patient;
using the gyroscope to obtain position measurements of the patient;
using the pressure sensor to obtain pressure measurements of air within the air passage;
using the processor and the pressure measurements to calculate respiratory air flow of the patient;
using the ammeter to obtain electrical current measurements of the PAP system;
using the processor and the electrical current measurements to detect a mask leak; and
analyzing the pressure measurements, heart rate measurements, oxygen saturation measurements, sound measurements, position measurements, and electrical current measurements concurrently to determine a sleep diagnosis, wherein:
a) a mask leak is detected,
and wherein the heart rate measurements are within a normal range,
and wherein the oxygen saturation measurements are within a normal range,
and wherein the sound measurements are within a normal range,
and wherein the position measurements are within a normal range,
whereby the diagnosis that is determined by the PAP system is that the patient does not require PAP treatment; and/or
b) a mask leak is detected,
and wherein the heart rate measurements are within a normal range,
and wherein the oxygen saturation measurements are not within a normal range,
and wherein the sound measurements are not within a normal range,
and wherein the position measurements are within a normal range,
whereby the diagnosis that is determined by the PAP system is that a determination needs to be made as to whether the mask fits securely on the patient's face; and/or
c) no mask leak is detected,
and wherein the heart rate measurements are not within a normal range,
and wherein the oxygen saturation measurements are within a normal range,
and wherein the sound measurements are within a normal range, and wherein the position measurements are not within a normal range,
whereby the diagnosis that is determined by the PAP system is that the mask needs to be re-fitted to the patient.

8. The non-volatile storage media of claim 7, wherein the processor uses the pressure measurements to detect the mask leak.

9. The non-volatile storage media of claim 7 in combination with claim 3, the method comprising:
using the at least one ECG position sensor to obtain ECG position measurements of the at least one ECG sensor;
using the processor and the ECG position measurements to calculate respiratory effort of the patient; and
analyzing the respiratory effort of the patient concurrently with the pressure measurements, heart rate measurements, oxygen saturation measurements, sound measurements, position measurements, and electrical current measurements to determine the sleep diagnosis.

10. The non-volatile storage media of claim 7, wherein a mask leak is detected,
and wherein the heart rate measurements are within a normal range,
and wherein the oxygen saturation measurements are not within a normal range,
and wherein the sound measurements are within a normal range,
and wherein the position measurements are not within a normal range,
whereby the diagnosis that is determined by the PAP system is that patient suffers from positional obstructive sleep apnea; and/or
The non-volatile storage media of claim 7, wherein no mask leak is detected,
and wherein the heart rate measurements are not within a normal range,
and wherein the oxygen saturation measurements are not within a normal range,
and wherein the sound measurements are within a normal range,
and wherein the position measurements are within a normal range,
whereby the diagnosis that is determined by the PAP system is that the heart rate monitor and pulse oximeter are not functioning as intended.

11. The non-volatile storage media of claim 7, wherein a mask leak is detected,
and wherein the heart rate measurements are within a normal range,
and wherein the oxygen saturation measurements are within a normal range,
and wherein the sound measurements are not within a normal range,
and wherein the position measurements are within a normal range,
whereby the diagnosis that is determined by the PAP system is that the patient suffers from mild obstructive sleep apnea and does not require PAP treatment; and/or

12. The non-volatile storage media of claim 7, wherein no mask leak is detected,
and wherein the heart rate measurements are within a normal range,
and wherein the oxygen saturation measurements are within a normal range,
and wherein the sound measurements are not within a normal range,
and wherein the position measurements are not within a normal range,
whereby the diagnosis that is determined by the PAP system is that the patient requires further sleep studies.

13. The non-volatile storage media of claim 7for performing a polysomnography using the PAP system of claim 1, the method comprising:
analyzing the pressure measurements, heart rate measurements, oxygen saturation measurements, sound measurements, position measurements, and electrical current measurements concurrently over a period of time to calculate an initial patient AHI;
analyzing the pressure measurements, heart rate measurements, oxygen saturation measurements, sound measurements, position measurements, and electrical current measurements concurrently over a period of time to calculate ongoing patient AHIs; and
analyzing the pressure measurements, heart rate measurements, oxygen saturation measurements, sound measurements, position measurements, and electrical current measurements concurrently to provide a diagnosis.

14. The non-volatile storage media of claim 13 in combination with claim 3,
the method further comprising:
using the at least one ECG position sensor to obtain ECG position measurements of the at least one ECG sensor;
using the processor and the ECG position measurements to calculate respiratory effort of the patient;
analyzing the respiratory effort of the patient concurrently with the pressure measurements, heart rate measurements, oxygen saturation measurements, sound measurements, position measurements, and electrical current measurements over a period of time to calculate the initial patient AHI;
analyzing the respiratory effort of the patient concurrently with the pressure measurements, heart rate measurements, oxygen saturation measurements, sound measurements, position measurements, and electrical current measurements concurrently over a period of time to calculate ongoing patient AHIs; and
analyzing the respiratory effort of the patient concurrently with the pressure measurements, heart rate measurements, oxygen saturation measurements, sound measurements, position measurements, and electrical current measurements concurrently to provide a diagnosis; and/or
The non-volatile storage media of claim 13, wherein the ongoing patient AHIs are lower than the initial patient AHI,
and wherein no mask leak is detected,
and wherein the heart rate measurements are within a normal range,
and wherein the oxygen saturation measurements are within a normal range,
and wherein the sound measurements are not within a normal range,
and wherein the position measurements are within a normal range,
whereby the diagnosis that is determined by the PAP system is that the patient requires surgery to treat obstructive sleep apnea.

15. The non-volatile storage media of claim 13, wherein the ongoing patient AHIs are lower than the initial patient AHI,
and wherein a mask leak is detected,
and wherein the heart rate measurements are within a normal range,
and wherein the oxygen saturation measurements are within a normal range,
and wherein the sound measurements are not within a normal range,
and wherein the position measurements are within a normal range,
whereby the diagnosis that is determined by the PAP system is that the patient is better suited for BiPAP treatment rather than CPAP treatment.
The method of claim 13, wherein the ongoing patient AHIs are lower than the initial patient AHI,
and wherein the ongoing patient AHIs are calculated to be 0,
and wherein no mask leak is detected,
and wherein the heart rate measurements are within a normal range,
and wherein the oxygen saturation measurements are within a normal range,
and wherein the sound measurements are within a normal range,
and wherein the position measurements are within a normal range,
whereby the diagnosis that is determined by the PAP system is that the PAP treatment is working as intended; and/or, wherein the ongoing patient AHIs are calculated as between 0 and 25,
and wherein a mask leak is detected,
and wherein the heart rate measurements are within a normal range,
and wherein the oxygen saturation measurements are not within a normal range,
and wherein the sound measurements are not within a normal range,
and wherein the position measurements are within a normal range,
and wherein the respiratory effort of the patient is not within a normal range,
and wherein the respiratory air flow of the patient is not within a normal range,
whereby the diagnosis that is determined by the PAP system is that the patient requires a better-fitting mask for the PAP system; and/or
, wherein the ongoing patient AHIs are calculated to be greater than 60,
and wherein a mask leak is detected,
and wherein the heart rate measurements are not within a normal range,
and wherein the oxygen saturation measurements are not within a normal range,
and wherein the sound measurements are not within a normal range,
and wherein the position measurements are within a normal range,
and wherein the respiratory effort of the patient is not within a normal range,
and wherein the respiratory air flow of the patient is not within a normal range,
whereby the diagnosis that is determined by the PAP system is that the patient requires a better-fitting mask for the PAP system; and/or
wherein the ongoing patient AHIs are calculated to be greater than 60,
and wherein a mask leak is detected,
and wherein the heart rate measurements are not within a normal range,
and wherein the oxygen saturation measurements are not within a normal range,
and wherein the sound measurements are not within a normal range,
and wherein the position measurements are within a normal range,
and wherein the respiratory effort of the patient is not within a normal range,
and wherein the respiratory air flow of the patient is not within a normal range,
whereby the diagnosis that is determined by the PAP system is that the patient is better suited for BiPAP treatment rather than CPAP treatment; and/or wherein the ongoing patient AHIs are calculated to be greater than 60,
and wherein a mask leak is detected,
and wherein the heart rate measurements are not within a normal range,
and wherein the oxygen saturation measurements are not within a normal range,
and wherein the sound measurements are not within a normal range,
and wherein the position measurements are within a normal range,
and wherein the respiratory effort of the patient is not within a normal range,
and wherein the respiratory air flow of the patient is not within a normal range,
whereby the diagnosis that is determined by the PAP system is that the PAP system should be adjusted to increase air pressure within the mask.

## Patentansprüche

1. PAP-System (10), umfassend:
eine PAP-Einheit (50), umfassend:
ein Gebläse (52);
einen Luftdurchgang (42), der zwischen der PAP-Einheit und dem Gesicht eines Patienten konfiguriert ist;
eine Maske (40);
einen Prozessor (32);
einen Herzfrequenzmonitor (20), wobei der Herzfrequenzmonitor Herzfrequenzmessungen des Patienten erfasst;
ein Pulsoximeter (21), wobei das Pulsoximeter Sauerstoffsättigungsmessungen des Patienten erfasst;
ein Mikrofon (24), wobei das Mikrofon Schallmessungen des Patienten erfasst;
ein Gyroskop (23), wobei das Gyroskop Positionsmessungen des Patienten erfasst;
einen Drucksensor (22), wobei der Drucksensor Druckmessungen der Luft innerhalb des Luftdurchgangs erfasst, wobei der Prozessor die Druckmessungen verwendet, um den Atemluftstrom des Patienten zu berechnen; und
einen Amperemesser (26), wobei der Amperemesser elektrische Strommessungen des PAP-Systems erfasst, wobei der Prozessor die elektrischen Strommessungen verwendet, um ein Maskenleck zu erkennen,
wobei die Druckmessungen, Herzfrequenzmessungen, Sauerstoffsättigungsmessungen, Schallmessungen, Positionsmessungen und elektrischen Strommessungen gleichzeitig vom Prozessor analysiert werden, um eine Schlafdiagnose zu bestimmen
wobei:
a) ein Maskenleck erkannt wird,
und wobei die Herzfrequenzmessungen innerhalb eines normalen Bereichs liegen,
und wobei die Sauerstoffsättigungsmessungen innerhalb eines normalen Bereichs liegen,
und wobei die Schallmessungen innerhalb eines normalen Bereichs liegen,
und wobei die Positionsmessungen innerhalb eines normalen Bereichs liegen,
wobei die vom PAP-System ermittelte Diagnose lautet, dass der Patient keine PAP-Behandlung benötigt; und/oder
b) ein Maskenleck erkannt wird,
wobei die Herzfrequenzmessungen innerhalb eines normalen Bereichs liegen,
und wobei die Sauerstoffsättigungsmessungen nicht innerhalb eines normalen Bereichs liegen,
und wobei die Schallmessungen nicht innerhalb eines normalen Bereichs liegen,
und wobei die Positionsmessungen innerhalb eines normalen Bereichs liegen,
wobei die vom PAP-System ermittelte Diagnose lautet, dass festgestellt werden muss, ob die Maske sicher auf dem Gesicht des Patienten sitzt; und/oder
c) wobei kein Maskenleck erkannt wird,
und wobei die Herzfrequenzmessungen nicht innerhalb eines normalen Bereichs liegen,
und wobei die Sauerstoffsättigungsmessungen innerhalb eines normalen Bereichs liegen,
und wobei die Schallmessungen innerhalb eines normalen Bereichs liegen,
und wobei die Positionsmessungen nicht innerhalb eines normalen Bereichs liegen,
wobei die vom PAP-System ermittelte Diagnose lautet, dass die Maske dem Patienten neu angepasst werden muss.

2. PAP-System nach Anspruch 1, wobei der Prozessor die Druckmessungen verwendet, um das Maskenleck zu erkennen.

3. PAP-System nach Anspruch 1, ferner umfassend:
mindestens einen ECG-Sensor (25); und
mindestens einen ECG-Positionssensor, der an jedem der mindestens einen ECG-Sensoren konfiguriert ist,
wobei der mindestens eine ECG-Positionssensor ECG-Positionsmessungen des mindestens einen ECG-Sensors erfasst,
wobei der Prozessor die ECG-Positionsmessungen verwendet, um die Atemanstrengung des Patienten zu berechnen,
und wobei die Atemanstrengung gleichzeitig mit den Druckmessungen, Herzfrequenzmessungen, Sauerstoffsättigungsmessungen, Schallmessungen, Positionsmessungen und elektrischen Strommessungen analysiert wird, um die Schlafdiagnose zu bestimmen.

4. PAP-System nach Anspruch 1, wobei ein Maskenleck erkannt wird,
und wobei die Herzfrequenzmessungen innerhalb eines normalen Bereichs liegen,
und wobei die Sauerstoffsättigungsmessungen nicht innerhalb eines normalen Bereichs liegen,
und wobei die Schallmessungen innerhalb eines normalen Bereichs liegen,
und wobei die Positionsmessungen nicht innerhalb eines normalen Bereichs liegen,
wobei die vom PAP-System ermittelte Diagnose lautet, dass der Patient an lagebedingter obstruktiver Schlafapnoe leidet; und/oder das PAP-System nach Anspruch 1, wobei kein Maskenleck erkannt wird,
und wobei die Herzfrequenzmessungen nicht innerhalb eines normalen Bereichs liegen,
und wobei die Sauerstoffsättigungsmessungen nicht innerhalb eines normalen Bereichs liegen,
und wobei die Schallmessungen innerhalb eines normalen Bereichs liegen,
und wobei die Positionsmessungen innerhalb eines normalen Bereichs liegen,
wobei die vom PAP-System ermittelte Diagnose lautet, dass der Herzfrequenzmonitor und das Pulsoximeter nicht wie vorgesehen funktionieren.

5. PAP-System nach Anspruch 1, wobei ein Maskenleck erkannt wird,
und wobei die Herzfrequenzmessungen innerhalb eines normalen Bereichs liegen,
und wobei die Sauerstoffsättigungsmessungen innerhalb eines normalen Bereichs liegen,
und wobei die Schallmessungen nicht innerhalb eines normalen Bereichs liegen,
und wobei die Positionsmessungen innerhalb eines normalen Bereichs liegen, wobei die vom PAP-System ermittelte Diagnose lautet, dass der Patient an leichter obstruktiver Schlafapnoe leidet und keine PAP-Behandlung benötigt; und/oder PAP-System nach Anspruch 1, wobei kein Maskenleck erkannt wird,
und wobei die Herzfrequenzmessungen innerhalb eines normalen Bereichs liegen,
und wobei die Sauerstoffsättigungsmessungen innerhalb eines normalen Bereichs liegen,
und wobei die Schallmessungen nicht innerhalb eines normalen Bereichs liegen,
und wobei die Positionsmessungen nicht innerhalb eines normalen Bereichs liegen,
wobei die vom PAP-System ermittelte Diagnose lautet, dass der Patient weitere Schlafstudien benötigt.

6. PAP-System nach Anspruch 1, wobei das Gyroskop auf einem tragbaren Artikel (70, 70') konfiguriert ist,
und wobei der Drucksensor innerhalb des Luftdurchgangs konfiguriert ist,
und wobei der Prozessor und der Amperemesser innerhalb der PAP-Einheit konfiguriert sind;
und/oder das PAP-System nach Anspruch 1, wobei der Herzfrequenzmonitor und das Pulsoximeter auf einem tragbaren Artikel konfiguriert sind, der vom Patienten getragen wird.

7. Nichtflüchtiges Speichermedium, das programmierbare Anweisungen speichert, die so konfiguriert sind, dass sie unter Verwendung des PAP-Systems nach einem der vorhergehenden Ansprüche ein Verfahren ausführen, wenn sie vom Prozessor ausgeführt werden, um unter Verwendung des PAP-Systems nach Anspruch 1 eine Diagnose durchzuführen, wobei das Verfahren umfasst:
Verwenden des Herzfrequenzmonitors, um Herzfrequenzmessungen eines Patienten zu erfassen;
Verwenden des Pulsoximeters, um Sauerstoffsättigungsmessungen des Patienten zu erfassen;
Verwenden des Mikrofons, um Schallmessungen des Patienten zu erfassen;
Verwenden des Gyroskops, um Positionsmessungen des Patienten zu erfassen;
Verwenden des Drucksensors, um Druckmessungen der Luft innerhalb des Luftdurchgangs zu erfassen;
Verwenden des Prozessors und der Druckmessungen, um den Atemluftstrom des Patienten zu berechnen;
Verwenden des Amperemessers, um elektrische Strommessungen des PAP-Systems zu erfassen;
Verwenden des Prozessors und der elektrischen Strommessungen, um ein Maskenleck zu erkennen; und
gleichzeitiges Analysieren der Druckmessungen, Herzfrequenzmessungen, Sauerstoffsättigungsmessungen, Schallmessungen, Positionsmessungen und elektrischen Strommessungen, um eine Schlafdiagnose zu bestimmen, wobei:
a) ein Maskenleck erkannt wird,
und wobei die Herzfrequenzmessungen innerhalb eines normalen Bereichs liegen,
und wobei die Sauerstoffsättigungsmessungen innerhalb eines normalen Bereichs liegen,
und wobei die Schallmessungen innerhalb eines normalen Bereichs liegen,
und wobei die Positionsmessungen innerhalb eines normalen Bereichs liegen,
wobei die vom PAP-System ermittelte Diagnose lautet, dass der Patient keine PAP-Behandlung benötigt; und/oder
b) ein Maskenleck erkannt wird,
und wobei die Herzfrequenzmessungen innerhalb eines normalen Bereichs liegen,
und wobei die Sauerstoffsättigungsmessungen nicht innerhalb eines normalen Bereichs liegen,
und wobei die Schallmessungen nicht innerhalb eines normalen Bereichs liegen,
und wobei die Positionsmessungen innerhalb eines normalen Bereichs liegen,
wobei die vom PAP-System ermittelte Diagnose lautet, dass festgestellt werden muss, ob die Maske sicher auf dem Gesicht des Patienten sitzt; und/oder
c) kein Maskenleck erkannt wird,
und wobei die Herzfrequenzmessungen nicht innerhalb eines normalen Bereichs liegen,
und wobei die Sauerstoffsättigungsmessungen innerhalb eines normalen Bereichs liegen,
und wobei die Schallmessungen innerhalb eines normalen Bereichs liegen,
und wobei die Positionsmessungen nicht innerhalb eines normalen Bereichs liegen,
wobei die vom PAP-System ermittelte Diagnose lautet, dass die Maske dem Patienten neu angepasst werden muss.

8. Nichtflüchtiges Speichermedium nach Anspruch 7, wobei der Prozessor die Druckmessungen verwendet, um das Maskenleck zu erkennen.

9. Nichtflüchtiges Speichermedium nach Anspruch 7 in Kombination mit Anspruch 3, wobei das Verfahren umfasst:
Verwenden des mindestens einen ECG-Positionssensors, um ECG-Positionsmessungen des mindestens einen ECG-Sensors zu erfassen;
Verwenden des Prozessors und der ECG-Positionsmessungen, um die Atemanstrengung des Patienten zu berechnen; und
gleichzeitiges Analysieren der Atemanstrengung des Patienten mit den Druckmessungen, Herzfrequenzmessungen, Sauerstoffsättigungsmessungen, Schallmessungen, Positionsmessungen und elektrischen Strommessungen, um die Schlafdiagnose zu bestimmen.

10. Nichtflüchtiges Speichermedium nach Anspruch 7, wobei ein Maskenleck erkannt wird,
und wobei die Herzfrequenzmessungen innerhalb eines normalen Bereichs liegen,
und wobei die Sauerstoffsättigungsmessungen nicht innerhalb eines normalen Bereichs liegen,
und wobei die Schallmessungen innerhalb eines normalen Bereichs liegen,
und wobei die Positionsmessungen nicht innerhalb eines normalen Bereichs liegen,
wobei die vom PAP-System ermittelte Diagnose lautet, dass der Patient an lagebedingter obstruktiver Schlafapnoe leidet; und/oder
Nichtflüchtiges Speichermedium nach Anspruch 7, wobei kein Maskenleck erkannt wird,
und wobei die Herzfrequenzmessungen nicht innerhalb eines normalen Bereichs liegen,
und wobei die Sauerstoffsättigungsmessungen nicht innerhalb eines normalen Bereichs liegen,
und wobei die Schallmessungen innerhalb eines normalen Bereichs liegen,
und wobei die Positionsmessungen innerhalb eines normalen Bereichs liegen,
wobei die vom PAP-System ermittelte Diagnose lautet, dass der Herzfrequenzmonitor und das Pulsoximeter nicht wie vorgesehen funktionieren.

11. Nichtflüchtiges Speichermedium nach Anspruch 7, wobei ein Maskenleck erkannt wird,
und wobei die Herzfrequenzmessungen innerhalb eines normalen Bereichs liegen,
und wobei die Sauerstoffsättigungsmessungen innerhalb eines normalen Bereichs liegen,
und wobei die Schallmessungen nicht innerhalb eines normalen Bereichs liegen,
und wobei die Positionsmessungen innerhalb eines normalen Bereichs liegen, wobei die vom PAP-System ermittelte Diagnose lautet, dass der Patient an leichter obstruktiver Schlafapnoe leidet und keine PAP-Behandlung benötigt; und/oder

12. Nichtflüchtiges Speichermedium nach Anspruch 7, wobei kein Maskenleck erkannt wird,
und wobei die Herzfrequenzmessungen innerhalb eines normalen Bereichs liegen,
und wobei die Sauerstoffsättigungsmessungen innerhalb eines normalen Bereichs liegen,
und wobei die Schallmessungen nicht innerhalb eines normalen Bereichs liegen,
und wobei die Positionsmessungen nicht innerhalb eines normalen Bereichs liegen,
wobei die vom PAP-System ermittelte Diagnose lautet, dass der Patient weitere Schlafstudien benötigt.

13. Nichtflüchtiges Speichermedium nach Anspruch 7 zum Durchführen einer Polysomnographie unter Verwendung des PAP-Systems nach Anspruch 1, wobei das Verfahren umfasst:
gleichzeitiges Analysieren der Druckmessungen, Herzfrequenzmessungen, Sauerstoffsättigungsmessungen, Schallmessungen, Positionsmessungen und elektrischen Strommessungen über einen bestimmten Zeitraum, um einen anfänglichen AHI des Patienten zu berechnen;
gleichzeitiges Analysieren der Druckmessungen, Herzfrequenzmessungen, Sauerstoffsättigungsmessungen, Schallmessungen, Positionsmessungen und elektrischen Strommessungen über einen bestimmten Zeitraum, um laufende AHI des Patienten zu berechnen; und
gleichzeitiges Analysieren der Druckmessungen, Herzfrequenzmessungen, Sauerstoffsättigungsmessungen, Schallmessungen, Positionsmessungen und elektrischen Strommessungen, um eine Diagnose bereitzustellen.

14. Nichtflüchtiges Speichermedium nach Anspruch 13 in Kombination mit Anspruch 3, wobei das Verfahren ferner umfasst:
Verwenden des mindestens einen ECG-Positionssensors, um ECG-Positionsmessungen des mindestens einen ECG-Sensors zu erfassen;
Verwenden des Prozessors und der ECG-Positionsmessungen, um die Atemanstrengung des Patienten zu berechnen;
gleichzeitiges Analysieren der Atemanstrengung des Patienten mit den Druckmessungen, Herzfrequenzmessungen, Sauerstoffsättigungsmessungen, Schallmessungen, Positionsmessungen und elektrischen Strommessungen über einen bestimmten Zeitraum, um den anfänglichen **AHI** des Patienten zu berechnen;
gleichzeitiges Analysieren der Atemanstrengung des Patienten mit den Druckmessungen, Herzfrequenzmessungen, Sauerstoffsättigungsmessungen, Schallmessungen, Positionsmessungen und elektrischen Strommessungen über einen bestimmten Zeitraum, um laufende **AHI** des Patienten zu berechnen; und
gleichzeitiges Analysieren der Atemanstrengung des Patienten mit den Druckmessungen, Herzfrequenzmessungen, Sauerstoffsättigungsmessungen, Schallmessungen, Positionsmessungen und elektrischen Strommessungen über einen bestimmten Zeitraum, um eine Diagnose bereitzustellen; und/oder Nichtflüchtige Speichermedium nach Anspruch 13, wobei die laufenden **AHI** des Patienten niedriger sind als die anfänglichen **AHI** des Patienten,
und wobei kein Maskenleck erkannt wird,
und wobei die Herzfrequenzmessungen innerhalb eines normalen Bereichs liegen,
und wobei die Sauerstoffsättigungsmessungen innerhalb eines normalen Bereichs liegen,
und wobei die Schallmessungen nicht innerhalb eines normalen Bereichs liegen,
und wobei die Positionsmessungen innerhalb eines normalen Bereichs liegen, wobei die vom PAP-System ermittelte Diagnose lautet, dass der Patient eine Operation zur Behandlung der obstruktiven Schlafapnoe benötigt.

15. Nichtflüchtige Speichermedium nach Anspruch 13, wobei die laufenden AHI des Patienten niedriger sind als die anfänglichen AHI des Patienten,
und wobei ein Maskenleck erkannt wird,
und wobei die Herzfrequenzmessungen innerhalb eines normalen Bereichs liegen,
und wobei die Sauerstoffsättigungsmessungen innerhalb eines normalen Bereichs liegen,
und wobei die Schallmessungen nicht innerhalb eines normalen Bereichs liegen,
und wobei die Positionsmessungen innerhalb eines normalen Bereichs liegen,
wobei die vom PAP-System ermittelte Diagnose lautet, dass für den Patienten eine BiPAP-Behandlung besser geeignet ist als eine CPAP-Behandlung.
Verfahren nach Anspruch 13, wobei die laufenden AHI des Patienten niedriger sind als die anfänglichen AHI des Patienten,
und wobei die laufenden AHI des Patienten mit 0 berechnet werden,
und wobei kein Maskenleck erkannt wird,
und wobei die Herzfrequenzmessungen innerhalb eines normalen Bereichs liegen,
und wobei die Sauerstoffsättigungsmessungen innerhalb eines normalen Bereichs liegen,
und wobei die Schallmessungen innerhalb eines normalen Bereichs liegen,
und wobei die Positionsmessungen innerhalb eines normalen Bereichs liegen,
wobei die vom PAP-System ermittelte Diagnose lautet, dass die PAP-Behandlung wie vorgesehen funktioniert; und/oder wobei die laufenden AHI des Patienten als Werte zwischen 0 und 25 berechnet werden,
und wobei ein Maskenleck erkannt wird,
und wobei die Herzfrequenzmessungen innerhalb eines normalen Bereichs liegen,
und wobei die Sauerstoffsättigungsmessungen nicht innerhalb eines normalen Bereichs liegen,
und wobei die Schallmessungen nicht innerhalb eines normalen Bereichs liegen,
und wobei die Positionsmessungen innerhalb eines normalen Bereichs liegen,
und wobei die Atemanstrengung des Patienten nicht innerhalb eines normalen Bereichs liegt,
und wobei der Atemluftstrom des Patienten nicht innerhalb eines normalen Bereichs liegt, wobei die vom PAP-System ermittelte Diagnose lautet, dass der Patient eine besser sitzende Maske für das PAP-System benötigt; und/oder
wobei die laufenden AHI des Patienten mit über 60 berechnet werden,
und wobei ein Maskenleck erkannt wird,
und wobei die Herzfrequenzmessungen nicht innerhalb eines normalen Bereichs liegen,
und wobei die Sauerstoffsättigungsmessungen nicht innerhalb eines normalen Bereichs liegen,
und wobei die Schallmessungen nicht innerhalb eines normalen Bereichs liegen,
und wobei die Positionsmessungen innerhalb eines normalen Bereichs liegen,
und wobei die Atemanstrengung des Patienten nicht innerhalb eines normalen Bereichs liegt,
und wobei der Atemluftstrom des Patienten nicht innerhalb eines normalen Bereichs liegt,
wobei die vom PAP-System ermittelte Diagnose lautet, dass der Patient eine besser sitzende Maske für das PAP-System benötigt; und/oder
wobei die laufenden AHI des Patienten mit über 60 berechnet werden,
und wobei ein Maskenleck erkannt wird,
und wobei die Herzfrequenzmessungen nicht innerhalb eines normalen Bereichs liegen,
und wobei die Sauerstoffsättigungsmessungen nicht innerhalb eines normalen Bereichs liegen,
und wobei die Schallmessungen nicht innerhalb eines normalen Bereichs liegen,
und wobei die Positionsmessungen innerhalb eines normalen Bereichs liegen,
und wobei die Atemanstrengung des Patienten nicht innerhalb eines normalen Bereichs liegt,
und wobei der Atemluftstrom des Patienten nicht innerhalb eines normalen Bereichs liegt,
wobei die vom PAP-System ermittelte Diagnose lautet, dass für den Patienten eine BiPAP-Behandlung besser geeignet ist als eine CPAP-Behandlung; und/oder
wobei die laufenden AHI des Patienten mit über 60 berechnet werden,
und wobei ein Maskenleck erkannt wird,
und wobei die Herzfrequenzmessungen nicht innerhalb eines normalen Bereichs liegen,
und wobei die Sauerstoffsättigungsmessungen nicht innerhalb eines normalen Bereichs liegen,
und wobei die Schallmessungen nicht innerhalb eines normalen Bereichs liegen,
und wobei die Positionsmessungen innerhalb eines normalen Bereichs liegen,
und wobei die Atemanstrengung des Patienten nicht innerhalb eines normalen Bereichs liegt,
und wobei der Atemluftstrom des Patienten nicht innerhalb eines normalen Bereichs liegt,
wobei die vom PAP-System ermittelte Diagnose lautet, dass das PAP-System angepasst werden sollte, um den Luftdruck in der Maske zu erhöhen.

## Revendications

1. Système PAP (10) comprenant :
une unité PAP (50) comprenant :
un ventilateur soufflant (52) ;
un passage d'air (42) configuré entre l'unité PAP et le visage d'un patient ;
un masque (40) ;
un processeur (32) ;
un moniteur de fréquence cardiaque (20), dans lequel le moniteur de fréquence cardiaque obtient des mesures de fréquence cardiaque du patient ;
un oxymètre de pouls (21), dans lequel l'oxymètre de pouls obtient des mesures de saturation en oxygène du patient ;
un microphone (24), dans lequel le microphone obtient des mesures acoustiques du patient ;
un gyroscope (23), dans lequel le gyroscope obtient des mesures de position du patient
un capteur de pression (22), dans lequel le capteur de pression obtient des mesures de pression d'air dans le passage d'air, dans lequel le processeur utilise lesdites mesures de pression pour calculer le débit d'air respiratoire du patient ; et
un ampèremètre (26), dans lequel l'ampèremètre obtient des mesures de courant électrique du système PAP, dans lequel le processeur utilise lesdites mesures de courant électrique pour détecter une fuite du masque,
dans lequel les mesures de pression, les mesures de fréquence cardiaque, les mesures de saturation en oxygène, les mesures acoustiques, les mesures de position et les mesures de courant électrique sont analysées simultanément par le processeur pour établir un diagnostic du sommeil,
dans lequel
a) une fuite du masque est détectée,
et dans lequel les mesures de fréquence cardiaque sont dans une plage normale,
et dans lequel les mesures de saturation en oxygène sont dans une plage normale,
et dans lequel les mesures acoustiques sont dans une plage normale,
et dans lequel les mesures de position sont dans une plage normale,
moyennant quoi le diagnostic qui est établi par le système PAP est que le patient n'a pas besoin de traitement PAP ; et/ou
b) une fuite du masque est détectée,
dans lequel les mesures de fréquence cardiaque sont dans une plage normale,
et dans lequel les mesures de saturation en oxygène ne sont pas dans une plage normale,
et dans lequel les mesures acoustiques ne sont pas dans une plage normale,
et dans lequel les mesures de position sont dans une plage normale,
moyennant quoi le diagnostic qui est établi par le système PAP est qu'il faut déterminer si le masque s'adapte fermement au visage du patient ; et/ou
c) dans lequel aucune fuite du masque n'est détectée,
et dans lequel les mesures de fréquence cardiaque ne sont pas dans une plage normale,
et dans lequel les mesures de saturation en oxygène sont dans une plage normale,
et dans lequel les mesures acoustiques sont dans une plage normale,
et dans lequel les mesures de position ne sont pas dans une plage normale,
moyennant quoi le diagnostic qui est établi par le système PAP est qu'il faut réadapter le masque au patient.

2. Système PAP selon la revendication 1, dans lequel le processeur utilise les mesures de pression pour détecter la fuite du masque.

3. Système PAP selon la revendication 1, comprenant en outre :
au moins un capteur ECG (25) ; et
au moins un capteur de position ECG configuré sur chacun des au moins un capteur ECG,
dans lequel l'au moins un capteur de position ECG obtient des mesures de position ECG de l'au moins un capteur ECG,
dans lequel le processeur utilise les mesures de position ECG pour calculer l'effort respiratoire du patient,
et dans lequel l'effort respiratoire est analysé simultanément avec les mesures de pression, les mesures de fréquence cardiaque, les mesures de saturation en oxygène, les mesures acoustiques, les mesures de position et les mesures de courant électrique pour établir le diagnostic du sommeil.

4. Système PAP selon la revendication 1, dans lequel une fuite du masque est détectée,
et dans lequel les mesures de fréquence cardiaque sont dans une plage normale,
et dans lequel les mesures de saturation en oxygène ne sont pas dans une plage normale,
et dans lequel les mesures acoustiques sont dans une plage normale,
et dans lequel les mesures de position ne sont pas dans une plage normale,
moyennant quoi le diagnostic qui est établi par le système PAP est que le patient souffre d'apnée obstructive du sommeil positionnelle ; et/ou
le système PAP selon la revendication 1, dans lequel aucune fuite du masque n'est détectée,
et dans lequel les mesures de fréquence cardiaque ne sont pas dans une plage normale,
et dans lequel les mesures de saturation en oxygène ne sont pas dans une plage normale,
et dans lequel les mesures acoustiques sont dans une plage normale,
et dans lequel les mesures de position sont dans une plage normale,
moyennant quoi le diagnostic qui est établi par le système PAP est que le moniteur de fréquence cardiaque et l'oxymètre de pouls ne fonctionnent pas comme prévu.

5. Système PAP selon la revendication 1, dans lequel une fuite du masque est détectée,
et dans lequel les mesures de fréquence cardiaque sont dans une plage normale,
et dans lequel les mesures de saturation en oxygène sont dans une plage normale,
et dans lequel les mesures acoustiques ne sont pas dans une plage normale,
et dans lequel les mesures de position sont dans une plage normale,
moyennant quoi le diagnostic qui est établi par le système PAP est que le patient souffre d'apnée obstructive du sommeil légère et n'a pas besoin de traitement PAP ; et/ou
le système PAP selon la revendication 1, dans lequel aucune fuite du masque n'est détectée,
et dans lequel les mesures de fréquence cardiaque sont dans une plage normale,
et dans lequel les mesures de saturation en oxygène sont dans une plage normale,
et dans lequel les mesures acoustiques ne sont pas dans une plage normale,
et dans lequel les mesures de position ne sont pas dans une plage normale,
moyennant quoi le diagnostic qui est établi par le système PAP est que le patient nécessite des études de sommeil supplémentaires.

6. Système PAP selon la revendication 1, dans lequel le gyroscope est configuré sur un vêtement (70, 70'),
et dans lequel le capteur de pression est configuré dans le passage d'air,
et dans lequel le processeur et l'ampèremètre sont configurés dans l'unité PAP.
et/ou le système PAP selon la revendication 1, dans lequel le moniteur de fréquence cardiaque et l'oxymètre de pouls sont configurés sur un vêtement porté par le patient.

7. Support de stockage non-volatil stockant des instructions programmables qui sont configurées pour exécuter un procédé utilisant le système PAP selon l'une des revendications précédentes lorsqu'elles sont exécutées par le processeur pour effectuer un diagnostic en utilisant le système PAP selon la revendication 1, le procédé comprenant :
l'utilisation du moniteur de fréquence cardiaque pour obtenir des mesures de fréquence cardiaque d'un patient ;
l'utilisation de l'oxymètre de pouls pour obtenir des mesures de saturation en oxygène du patient ;
l'utilisation du microphone pour obtenir des mesures acoustiques du patient ;
l'utilisation du gyroscope pour obtenir des mesures de position du patient ;
l'utilisation du capteur de pression pour obtenir des mesures de pression d'air dans le passage d'air ;
l'utilisation du processeur et des mesures de pression pour calculer le débit d'air respiratoire du patient ;
l'utilisation de l'ampèremètre pour obtenir des mesures de courant électrique du système PAP ;
l'utilisation du processeur et des mesures de courant électrique pour détecter une fuite du masque ; et
l'analyse des mesures de pression, des mesures de fréquence cardiaque, des mesures de saturation en oxygène, des mesures acoustiques, des mesures de position et des mesures de courant électrique simultanément pour établir un diagnostic du sommeil, dans lequel :
a) une fuite du masque est détectée,
et dans lequel les mesures de fréquence cardiaque sont dans une plage normale,
et dans lequel les mesures de saturation en oxygène sont dans une plage normale,
et dans lequel les mesures acoustiques sont dans une plage normale,
et dans lequel les mesures de position sont dans une plage normale,
moyennant quoi le diagnostic qui est établi par le système PAP est que le patient n'a pas besoin de traitement PAP ; et/ou
b) une fuite du masque est détectée,
et dans lequel les mesures de fréquence cardiaque sont dans une plage normale,
et dans lequel les mesures de saturation en oxygène ne sont pas dans une plage normale,
et dans lequel les mesures acoustiques ne sont pas dans une plage normale,
et dans lequel les mesures de position sont dans une plage normale,
moyennant quoi le diagnostic qui est établi par le système PAP est qu'il faut déterminer si le masque s'adapte fermement au visage du patient ; et/ou
c) aucune fuite du masque n'est détectée,
et dans lequel les mesures de fréquence cardiaque ne sont pas dans une plage normale,
et dans lequel les mesures de saturation en oxygène sont dans une plage normale,
et dans lequel les mesures acoustiques sont dans une plage normale,
et dans lequel les mesures de position ne sont pas dans une plage normale,
moyennant quoi le diagnostic qui est établi par le système PAP est qu'il faut réadapter le masque au patient.

8. Support de stockage non-volatil selon la revendication 7, dans lequel le processeur utilise les mesures de pression pour détecter la fuite du masque.

9. Support de stockage non-volatil selon la revendication 7 en combinaison avec la revendication 3, le procédé comprenant :
l'utilisation de l'au moins un capteur de position ECG pour obtenir des mesures de position ECG de l'au moins un capteur ECG ;
l'utilisation du processeur et des mesures de position ECG pour calculer l'effort respiratoire du patient ; et
l'analyse de l'effort respiratoire du patient en même temps que les mesures de pression, les mesures de fréquence cardiaque, les mesures de saturation en oxygène, les mesures acoustiques, les mesures de position et les mesures de courant électrique pour établir le diagnostic du sommeil.

10. Support de stockage non-volatil selon la revendication 7, dans lequel une fuite du masque est détectée,
et dans lequel les mesures de fréquence cardiaque sont dans une plage normale,
et dans lequel les mesures de saturation en oxygène ne sont pas dans une plage normale,
et dans lequel les mesures acoustiques sont dans une plage normale,
et dans lequel les mesures de position ne sont pas dans une plage normale,
moyennant quoi le diagnostic qui est établi par le système PAP est que le patient souffre d'apnée obstructive du sommeil positionnelle ; et/ou
Support de stockage non-volatil selon la revendication 7, dans lequel aucune fuite du masque n'est détectée,
et dans lequel les mesures de fréquence cardiaque ne sont pas dans une plage normale,
et dans lequel les mesures de saturation en oxygène ne sont pas dans une plage normale,
et dans lequel les mesures acoustiques sont dans une plage normale,
et dans lequel les mesures de position sont dans une plage normale,
moyennant quoi le diagnostic qui est établi par le système PAP est que le moniteur de fréquence cardiaque et l'oxymètre de pouls ne fonctionnent pas comme prévu.

11. Support de stockage non-volatil selon la revendication 7, dans lequel une fuite du masque est détectée,
et dans lequel les mesures de fréquence cardiaque sont dans une plage normale,
et dans lequel les mesures de saturation en oxygène sont dans une plage normale,
et dans lequel les mesures acoustiques ne sont pas dans une plage normale,
et dans lequel les mesures de position sont dans une plage normale,
moyennant quoi le diagnostic qui est établi par le système PAP est que le patient souffre d'apnée obstructive du sommeil légère et n'a pas besoin de traitement PAP ; et/ou

12. Support de stockage non-volatil selon la revendication 7, dans lequel aucune fuite du masque n'est détectée,
et dans lequel les mesures de fréquence cardiaque sont dans une plage normale,
et dans lequel les mesures de saturation en oxygène sont dans une plage normale,
et dans lequel les mesures acoustiques ne sont pas dans une plage normale,
et dans lequel les mesures de position ne sont pas dans une plage normale,
moyennant quoi le diagnostic qui est établi par le système PAP est que le patient nécessite des études de sommeil supplémentaires.

13. Support de stockage non-volatil selon la revendication 7 pour effectuer une polysomnographie en utilisant le système PAP selon la revendication 1, le procédé comprenant :
l'analyse des mesures de pression, des mesures de fréquence cardiaque, des mesures de saturation en oxygène, des mesures acoustiques, des mesures de position et des mesures de courant électrique simultanément sur une période de temps donnée pour calculer un **IAH** initial du patient ;
l'analyse des mesures de pression, des mesures de fréquence cardiaque, des mesures de saturation en oxygène, des mesures acoustiques, des mesures de position et des mesures de courant électrique simultanément sur une période de temps donnée pour calculer les **IAH en** cours des patients ; et
l'analyse des mesures de pression, des mesures de fréquence cardiaque, des mesures de saturation en oxygène, des mesures acoustiques, des mesures de position et des mesures de courant électrique simultanément pour établir un diagnostic.

14. Support de stockage non-volatil selon la revendication 13 en combinaison avec la revendication 3,
le procédé comprenant en outre :
l'utilisation de l'au moins un capteur de position ECG pour obtenir des mesures de position ECG de l'au moins un capteur ECG ;
l'utilisation du processeur et des mesures de position ECG pour calculer l'effort respiratoire du patient ;
l'analyse de l'effort respiratoire du patient simultanément avec les mesures de pression, les mesures de fréquence cardiaque, les mesures de saturation en oxygène, les mesures acoustiques, les mesures de position et les mesures de courant électrique sur une période de temps donnée pour calculer l'IAH initial du patient ;
l'analyse de l'effort respiratoire du patient simultanément avec les mesures de pression, les mesures de fréquence cardiaque, les mesures de saturation en oxygène, les mesures acoustiques, les mesures de position et les mesures de courant électrique simultanément sur une période de temps donnée pour calculer les IAH en cours du patient ; et
l'analyse de l'effort respiratoire du patient simultanément avec les mesures de pression, les mesures de fréquence cardiaque, les mesures de saturation en oxygène, les mesures acoustiques, les mesures de position et les mesures de courant électrique simultanément pour établir un diagnostic ; et/ou
Support de stockage non-volatil selon la revendication 13, dans lequel les IAH en cours du patient sont inférieurs à l'IAH initial du patient,
et dans lequel aucune fuite du masque n'est détectée,
et dans lequel les mesures de fréquence cardiaque sont dans une plage normale,
et dans lequel les mesures de saturation en oxygène sont dans une plage normale,
et dans lequel les mesures acoustiques ne sont pas dans une plage normale,
et dans lequel les mesures de position sont dans une plage normale,
moyennant quoi le diagnostic qui est établi par le système PAP est que le patient a besoin d'une intervention chirurgicale pour traiter l'apnée obstructive du sommeil.

15. Support de stockage non-volatil selon la revendication 13, dans lequel les IAH en cours du patient sont inférieurs à l'IAH initial du patient,
et dans lequel une fuite du masque est détectée,
et dans lequel les mesures de fréquence cardiaque sont dans une plage normale,
et dans lequel les mesures de saturation en oxygène sont dans une plage normale,
et dans lequel les mesures acoustiques ne sont pas dans une plage normale,
et dans lequel les mesures de position sont dans une plage normale,
moyennant quoi le diagnostic qui est établi par le système PAP est que le patient a besoin d'un traitement BiPAP plutôt qu'au traitement CPAP.
Procédé selon la revendication 13, dans lequel les IAH en cours du patient sont inférieurs à l'IAH initial du patient,
et dans lequel les IAH en cours du patient sont calculés comme étant égaux à 0,
et dans lequel aucune fuite du masque n'est détectée,
et dans lequel les mesures de fréquence cardiaque sont dans une plage normale,
et dans lequel les mesures de saturation en oxygène sont dans une plage normale,
et dans lequel les mesures acoustiques sont dans une plage normale,
et dans lequel les mesures de position sont dans une plage normale,
moyennant quoi le diagnostic qui est établi par le système PAP est que le traitement PAP fonctionne comme prévu ; et/ou dans lequel les IAH en cours du patient sont calculés comme étant compris entre 0 et 25,
et dans lequel une fuite du masque est détectée,
et dans lequel les mesures de fréquence cardiaque sont dans une plage normale,
et dans lequel les mesures de saturation en oxygène ne sont pas dans une plage normale,
et dans lequel les mesures acoustiques ne sont pas dans une plage normale,
et dans lequel les mesures de position sont dans une plage normale,
et dans lequel l'effort respiratoire du patient n'est pas dans une plage normale,
et dans lequel le débit d'air respiratoire du patient n'est pas dans une plage normale,
moyennant quoi le diagnostic qui est établi par le système PAP est que le patient a besoin d'un masque qui est mieux adapté pour le système PAP ; et/ou
dans lequel les IAH en cours du patient sont calculés comme étant supérieurs à 60,
et dans lequel une fuite du masque est détectée,
et dans lequel les mesures de fréquence cardiaque ne sont pas dans une plage normale,
et dans lequel les mesures de saturation en oxygène ne sont pas dans une plage normale,
et dans lequel les mesures acoustiques ne sont pas dans une plage normale,
et dans lequel les mesures de position sont dans une plage normale,
et dans lequel l'effort respiratoire du patient n'est pas dans une plage normale,
et dans lequel le débit d'air respiratoire du patient n'est pas dans une plage normale,
moyennant quoi le diagnostic qui est établi par le système PAP est que le patient a besoin d'un masque qui est mieux adapté pour le système PAP ; et/ou
dans lequel les IAH en cours du patient sont calculés comme étant supérieurs à 60,
et dans lequel une fuite du masque est détectée,
et dans lequel les mesures de fréquence cardiaque ne sont pas dans une plage normale,
et dans lequel les mesures de saturation en oxygène ne sont pas dans une plage normale,
et dans lequel les mesures acoustiques ne sont pas dans une plage normale,
et dans lequel les mesures de position sont dans une plage normale,
et dans lequel l'effort respiratoire du patient n'est pas dans une plage normale,
et dans lequel le débit d'air respiratoire du patient n'est pas dans une plage normale,
moyennant quoi le diagnostic qui est établi par le système PAP est que le patient a besoin d'un traitement BiPAP plutôt qu'un traitement CPAP ; et/ou
dans lequel les IAH en cours du patient sont calculés comme étant supérieurs à 60,
et dans lequel une fuite du masque est détectée,
et dans lequel les mesures de fréquence cardiaque ne sont pas dans une plage normale,
et dans lequel les mesures de saturation en oxygène ne sont pas dans une plage normale,
et dans lequel les mesures acoustiques ne sont pas dans une plage normale,
et dans lequel les mesures de position sont dans une plage normale,
et dans lequel l'effort respiratoire du patient n'est pas dans une plage normale,
et dans lequel le débit d'air respiratoire du patient n'est pas dans une plage normale,
moyennant quoi le diagnostic qui est établi par le système PAP est que le système PAP doit être ajusté pour augmenter la pression d'air à l'intérieur du masque.
